(19) Europäisches Patentamt
European Patent Office
Office européen des brevets

(11) EP 3 687 507 B1

(12) **EUROPEAN PATENT SPECIFICATION**

(45) Date of publication and mention of the grant of the patent:
**22.11.2023 Bulletin 2023/47**

(21) Application number: **18782910.6**

(22) Date of filing: **26.09.2018**

(51) International Patent Classification (IPC):
***A61K 9/51*** *(2006.01)* ***A61K 9/00*** *(2006.01)*
***A61K 9/10*** *(2006.01)*

(52) Cooperative Patent Classification (CPC):
**A61K 9/5153; A61K 9/0053; A61K 9/10; A61K 9/5146**

(86) International application number:
**PCT/EP2018/076171**

(87) International publication number:
**WO 2019/063638 (04.04.2019 Gazette 2019/14)**

(54) **FUNCTIONALIZED POLYMERS FOR THE INTRACELLULAR RELEASE OF BIOACTIVE MOLECULES**

FUNKTIONALISIERTE POLYMERE ZUR INTRAZELLULÄREN FREISETZUNG VON BIOAKTIVEN MOLEKÜLEN

POLYMÈRES FONCTIONNALISÉS POUR LA LIBÉRATION INTRACELLULAIRE DE MOLÉCULES BIOLOGIQUEMENT ACTIVES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2017 IT 201700108274**

(43) Date of publication of application:
**05.08.2020 Bulletin 2020/32**

(73) Proprietor: **Sunyamed S.R.L.**
**84122 Salerno (IT)**

(72) Inventors:
- **PELUSO, Gianfranco**
  **80131 Napoli (IT)**
- **CALARCO, Anna**
  **89058 Scilla (RC) (IT)**

(74) Representative: **Bianchetti & Minoja with Trevisan & Cuonzo IPS SRL**
**Via Plinio, 63**
**20129 Milano (IT)**

(56) References cited:
**CN-A- 107 176 911**

- **LONGFA KOU ET AL: "L-carnitine-conjugated nanoparticles to OCTN2 and ATB 0,+ to deliver chemotherapeutic agents for colon cancer therapy", DRUG DELIVERY., vol. 24, no. 1, 1 January 2017 (2017-01-01), pages 1338-1349, XP55534661, US ISSN: 1071-7544, DOI: 10.1080/10717544.2017.1377316**
- **LONGFA KOU ET AL: "Supporting information. Dual Targeting of L-carnitine-conjugated Nanoparticles to OCTN2 and ATB0,+ to Deliver Chemotherapeutic Agents for Colon Cancer Therapy.", FRONTIERS IN PHARMACOLOGY, vol. 9, 15 September 2017 (2017-09-15), pages S1-S10, XP55534666, CH ISSN: 1663-9812, DOI: 10.3389/fphar.2018.00027**
- **GAO Y ET AL: "Chitosan N-betainates/DNA self-assembly nanoparticles for gene delivery: In vitro uptake and transfection efficiency", INTERNATIONAL JOURNAL OF PHARMACEUTICS, ELSEVIER, NL, vol. 371, no. 1-2, 17 April 2009 (2009-04-17), pages 156-162, XP026071375, ISSN: 0378-5173, DOI: 10.1016/J.IJPHARM.2008.12.012 [retrieved on 2009-03-25]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **MEGAN ROTH ET AL: "OATPs, OATs and OCTs: the organic anion and cation transporters of the SLCO and SLC22A gene superfamilies : OATPs, OATs and OCTs", BRITISH JOURNAL OF PHARMACOLOGY, vol. 165, no. 5, 10 February 2012 (2012-02-10), pages 1260-1287, XP055480549, UK ISSN: 0007-1188, DOI: 10.1111/j.1476-5381.2011.01724.x**

## Description

This invention pertains to bioactive molecule delivery systems, such systems comprising a biocompatible and/or biodegradable polymer derivatized with radicals R, A, B or C described herein and their use for transporting bioactive compounds inside cells that express an organic cationic transporter, particularly SLC22A4, SLC6A14 and SLC22A16.

## Background

In recent years, many efforts have been made to develop formulations suitable for the administration of bioactive-either synthetic or natural-molecules, characterized by physical and/or chemical instability, susceptibility to enzymatic degradation, pharmacokinetics unfavourable pharmacodynamics, and/or poor bioavailability.

Currently, various devices, especially nanostructured ones, are being studied as potential drug delivery systems. Among these, remarkable polymer nanoparticles have been developed, produced from different types of polymers of both natural and synthetic origin (Pharmaceutical Technology Europe, 17: 4, 21-28, 2005). Among the most used polymers we find aliphatic polyesters such as polylactic acid (PLA) (Laroui H., et al., Biomacromolecules, 8: 3879- 3885, 2007), poly-glycolic acid (PLGA), polyhydroxybutyrate (PHB) and their respective copolymers (Makhlof A., et al., Eur J Pharm Biopharm, 72: 1-8, 2009; Meissner Y., et al., Int J Pharm, 316: 138-143, 2006). Such polymers are already used in humans and are characterized by a high biocompatibility and in some cases also by their biodegradability (Yang H., et al., J Nanosci Nanotechnol, 9: 282-287, 2009).

The polymeric nanoparticles, thanks to their profile, are able to protect the drug from enzymatic degradation, promote its diffusion through the epithelium, modify the pharmacokinetics and distribution in the tissues and increase intracellular penetration. They can be administered through all classic routes of administration and are able to increase both the bioavailability and the therapeutic efficacy of the transported drug. The factors that have hampered the use of polymeric nanoparticles in the current clinical practice have been the controversial results on the internalization of the same in cells and tissues. These discrepancies can be ascribed, at least in part, to the different polymer nanoparticles used, to different experimental conditions and in vitro and in vivo biological systems tested.

*De facto*, the key features for a release system to be suitable, lie in the penetration efficiency in the target tissues and thus in the science of the internalization mechanisms and the intracellular traffic.

The size of nanoparticles is an important feature with regard to the diffusion through biological barriers. It has been established by several studies that, following a mucosal application, the smaller the particle size, the greater the possibility that the nanoparticles transport the drug through the mucosa itself (Cheng G. et al., World J Gastroenterol 10: 1769-1774, 2004, Couvreur P, and Vauthier C. Pharmaceutical Research, 23: 1417-1450, 2006). Several studies have shown that nanoparticles are carried within most of the cells significantly more efficiently if they are around 100 nm in size compared to 1 $\mu$m particles. The dimensions also influence transcellular transport since nanoparticles with a diameter of around 100 nm are able to reach the submucosal zone in vivo, while the microparticles remain confined mainly in the superficial epithelium (Singh M, et al., Biotechnol Adv 20: 5-6, 341-359, 2002).

The plasma membrane of living cells regulates the transport of molecules inside and outside the cell. It has been hypothesized that ions and small molecules enter the cells by passively diffusing through the lipid double layer or using finely regulated membrane channels. Larger molecules enter cells with different mechanisms based on their ability to interact with the cell membrane due to surface charges before being internalized in general by endocytosis or by binding to surface structures that favor carrier-mediated transport to inside the cell (Avrvizo, RR, et al., Nano Lett. 2010, 10, 2543-2548).

A large number of endogenous metabolites as well as drugs and xenobiotics are positively charged organic molecules (cations) whose absorption, distribution and excretion are strongly dependent on the expression of transport systems for organic ions present on the cell membrane. There are two main superfamilies of transporters: solute carriers (solute carrier transporters, SLC) and cassette transporters (ATP-binding cassette, ABC). [Roth M, et al., British Journal of Pharmacology. 2012; 165 (5): 1260-1287].

Since the identification and characterization of the first SLC transporter in 1994 [Gründemann D, et al., Nature 1994, 372: 549-552] other family members have been cloned from various species including humans, many of which have also been functionally characterized [Koepsell H., Mol Aspects Med 2013, 34: 413-435]. For example, the SLC22 family comprises three subgroups: one for organic cations (OCT), one for organic cations and zwitterions (OCTN) and one for organic anions (OAT).

Amongst the OCTs, the transporters SLC22A4, SLC6A14 and SLC22A16, favor intracellular and / or transcellular transport [Nigam K.S. Nature reviews Drug Discovery 14: 29-44, 2015]. Mature coating epithelial cells such as enterocytes, respiratory mucosa cells and conjunctival cells are known to naturally express SLC6A14 and / or SLC22A4 [Koepsell H, et al., Pharm Res. 2007 Jul; 24 (7): 1227-51]. Moreover, some OCTs (especially SLC22A16 and SLC6A14) are preferentially expressed on tumor cells (Wu Y. et al., Apoptosis.20: 1099-108, 2015; Coothankandaswamy V. et al., J Pharmacol., 2016 doi: 10.1111 / bph. 13616), opening the possibility of exploiting these carriers to convey bioactive

agents within certain target cells. The exploitation of both SLC22A4 and SLC6A14 would be particularly advantageous in the case of tumor cells expressing low levels of SLC22A16 (Oguri T. et al., Biomed Rep. 5: 639-643, 2016).

**Prior art**

The functionalization of polymeric nanoparticles, with chemically bound residues or simply adsorbed to their surface, capable of giving the nanoparticle a positive or negative net charge has been proposed to increase the uptake of the nanoparticulate by target cells. In the case of nanoparticles with positive charge, their increased internalization appears to be due to the greater interaction of the nanoparticulate with the negatively charged plasma membrane. It would therefore be a nonspecific mechanism not linked to interactions with membrane transporters as has been comprehensively demonstrated by the saturation curves and the kinetics of internalization of the nanoparticles (Mailänder V. and Landfester K., Biomacromolecules 10: 2379-2400, 2009).

Polycationic synthetic polymers (e.g. polyethylenimine, poly-lysine, and polydimethylaminoethylmethacrylates), have been suggested for various applications in medicine, especially in the field of tissue engineering and gene therapy, but have been found not be highly suitable as they are known to trigger in vivo adverse reactions linked to intrinsic toxicity, poor hemocompatibility and poor biodegradability (Moreau E, et al., Drug, Target, 10: 161-173, 2002). Among the natural polycationic polymers, some success as a drug delivery platform was obtained with chitosan. Chitosan's high degree of swelling in aqueous environment implies however a rapid release of the active molecule before reaching the target tissue (Bhattarai N, et al., Adv Drug Deliv Rev., 62: 83-99, 2010; Park JH, et al., Deliv Rev., 62: 28-41, 2010).

Modifications of polymers through insertion of charged residues or through 'quaternization' of primary amines present in the starting polymer have also been proposed to increase the charge of the final polymer modulating its ability to interact with the plasma membrane. Such modifications, however, typically do not overcome the problem of aspecific binding and merely improve the drug release kinetics from the nanodevice and the ability of the nanoparticulate to cross the cell / cell junctions at the intestinal level and thus avoiding the more efficient but less achievable trans-cellular route, (Lv PP et al. Biomacromolecules, 12: 4230-4239, 2011).

Longfa Kou et al. (Drug Delivery, vol. 24, no.1, 2017, pages 1338-1349) discloses carnitine-conjugated poly(lactic-co-glycolic acid) (PLGA) nanoparticles for the delivery of chemotherapeutic drugs into cancer cells. The nanoparticles are prepared by mixing stearoyl-carnitine with PLGA followed by addition of the drug.

In this disclosure, carnitine is in the form of an ester carrying a stearyl residue at the 3-hydroxyl group.

CN107176911 discloses nanoparticles non-covalently charged with stearoyl-L-carnitine. The authors however fail to demonstrate that the uptake of such compounds occurs via the SLC22A5 (OCTN2) transporter.

Gao Y et al., (Int J Pharm, 371, no. 1-2, 2009, pages 156-162) disclose how N-betaine-derived chitosan nanoparticles are able to transfect Cos-7 cells. Cos-7 cells are known, however, not to express OCTs (Wu et al., Experimental and Therapeutic Medicine, 14, 4153-4159, 2017 and Kaewmogul et al., Am J Physiol Renal Physiol, 285, F1149-1159, 2003) and thus the import of functionalized chitosan nanoparticles is not mediated by OCT.

**Brief description of the figures**

**Fig. 1** Effect of PHB-$CTB_{20}$ nanodevices loaded with curcumin (1:20) on cell viability after 24, 48 e 72 hours incubation with HF (a), HEK293 (b) e HCjE (c) cells.

**Fig. 2**: Effect of PLGA-$AMC_{20}$ nanodevices loaded with indomethacin (1:10) on cell viability after 24, 48 e 72 hours incubation with HF (a), HEK293 (b) e HCjE (c) cells.

**Fig. 3**: Quantity of curcumin (a), indomethacin (b) and acyclovir (c) present in HEK293-A4, HEK293-A14 e HEK293-A16 after treatment for 1,3 and 8 hours with 100μg/ml of drug-loaded PHB-$CTB_{20}$.

**Fig. 4**: Quantity of curcumin (a), indomethacin (b) and acyclovir (c) present in HEK293-A4, HEK293-A14 e HEK293-A16 after treatment for 1,3 and 8 hours with 100μg/ml of drug-loaded PLGA-$AMC_{20}$.

**Fig. 5**. Quantity of coumarin-6 present in present in HEK293-A14 (A), HEK293-A4 (B), and HEK293-A16 (C) cells after treatment for 0,5,10,15,20 and 40 minutes with 10μg/ml of drug-loaded PLGA-$AMC_{20}$.

**Fig. 6**. Quantity of coumarin-6 present in present in) HEK293-A16 (a), HEK293-A4 (b) and HEK293-A14 (c) cells after treatment for 0,5,10,15,20 and 40 minutes with 10μg/ml of drug-loaded PHB-$CTB_{20}$

**Fig. 7**. Quantity of coumarin-6 present in present in present in HEK293-A16 (a), HEK293-A4 (b) and HEK293-A14 (c) cells after treatment for 0,5,10,15,20 and 40 minutes with 10μg/ml of drug-loaded PLGA-$TIC_{20}$

**Fig. 8**. Quantity of coumarin-6 present in present in HEK293-A14 (a), HEK293-A4 (b) and HEK293-A16 (c) cells after treatment for 0,5,10,15,20 and 40 minutes with 10μg/ml of drug-loaded PHB-$CAC_{20}$

**Fig. 9**. Quantity of coumarin-6 present in present in HEK293-A14 (a), HEK293-A4 (b) and HEK293-A16 (c) cells after treatment for 0,5,10,15,20 and 40 minutes with 10μg/ml of drug-loaded PLGA-$DIC_{20}$

**Fig. 10**: Quantity of curcumin (a), indomethacin (b) and acyclovir (c) present in HEK293-A4, HEK293-A14 e HEK293-A16 after treatment for 1,3 and 8 hours with 100$\mu$g/ml of drug-loaded PLGA-$CTI_{20}$.
**Fig. 11**. Quantity of coumarin-6 present in present in HEK293-A16 (a), HEK293-A4 (b) and HEK293-A14 (c) cells after treatment for 0,5,10,15,20 and 40 minutes with 10$\mu$g/ml of drug-loaded PLGA-$CTI_{20}$
**Fig. 12**. Quantity of coumarin-6 present in present in HEK293-A4 (a), HEK293-A14 (b) and HEK293-A16 (c) cells after treatment for 0,5,10,15,20 and 40 minutes with 10$\mu$g/ml of drug-loaded PLGA-$CAR_{20}$
**Fig. 13** Quantity of coumarin-6 present in present in HEK293-A4, HEK293-A14 e HEK293-A16 after treatment for 1,3 and 8 with 100$\mu$g/ml of drug-loaded PHB-$CTB_{20}$ (a), PHB-Lys 1:20 (b) o PHB-Chito (c).
**Fig. 14**: In vivo bioavailability of curcumin when administered in free form or incapsulated in PHB-CTB nanoparticle of the invention.
**Fig. 15**: In vivo bioavailability of TB when administered in free form or incapsulated in a PLGA-$AMC_{20}$ nanoparticle.

## Description of the invention

We have surprisingly found that polymers derivatized with specific trialkylammonium radicals are able, due to their interaction with OCTs, and in particular with SLC22A4, SLC6A14 and SLC22A16, to efficiently release bioactive compounds inside cells that express such transporters.

This invention thus provides a bioactive molecule delivery system comprising:

a) a bioactive molecule; and
b) a biocompatible and/or biodegradable polymer in the form of nanoparticles covalently derivatized with a radical of formula R

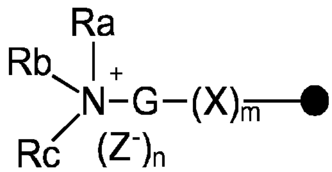

**R**

optionally via linker, and wherein:

● is the point of attachment to the polymer, or, when a linker is present, to the linker;
Ra, Rb and Rc can be the same or different and are selected from methyl and ethyl;
G is a $C_1$-$C_5$ linear, branched or cyclic alkyl, optionally substituted with one or more groups selected from hydroxy, carboxyl, carboxylate, 1-pyrrolidinyl, 1-azetidinyl, 1-aziridinyl, sulfhydryl, -NRdRe and -N=NRf;
Rd, Re and Rf are independently selected from hydrogen, methyl, ethyl and propyl; m can be zero or 1;
-X- is selected form the list of -C(O)-, -NRg-, -S- and $-SO_2-$,
Rg is selected from hydrogen, methyl, ethyl and propyl;
Z is a pharmaceutically acceptable anion;
n can be zero or 1, and it can only be zero when G bears a carboxylate;
with the proviso that R cannot be

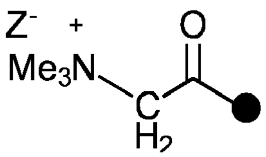

In one embodiment, G is a $C_1$-$C_2$ alkyl optionally substituted with one or more groups selected from hydroxyl, carboxyl, carboxylate, 1-pyrrolidinyl, 1-azetidinyl, 1-azirinidyl, sulfhydryl, NRdRe and -N=NRf.

In one embodiment, G is a $C_3$, cyclic, linear or branched alkyl group optionally substituted with one or more groups selected from hydroxyl, carboxyl, carboxylate, 1-pyrrolidinyl, 1-azetidinyl, 1-azirinidyl, sulfhydryl, NRdRe and -N=NRf.

In one embodiment, G is a $C_4$, cyclic, linear or branched alkyl group optionally substituted with one or more groups selected from hydroxyl, carboxyl, carboxylate, 1-pyrrolidinyl, 1-azetidinyl, 1-azirinidyl, sulfhydryl, NRdRe and

-N=NRf.

In one embodiment, G is a $C_5$, cyclic, linear or branched alkyl group optionally substituted with one or more groups selected from hydroxyl, carboxyl, carboxylate, 1-pyrrolidinyl, 1-azetidinyl, 1-azirinidyl, sulfhydryl, NRdRe and -N=NRf.

In one embodiment, R is selected from radicals, A, B and C

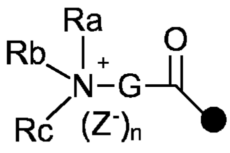

**A**

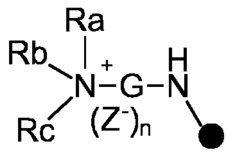

**B**

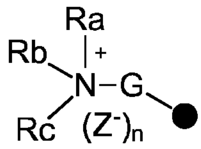

**C**

In one embodiment, R is selected from

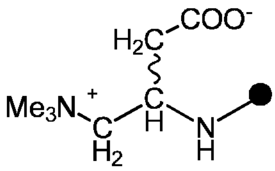
;
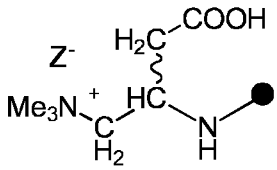
;

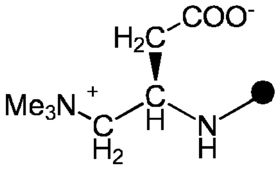
;
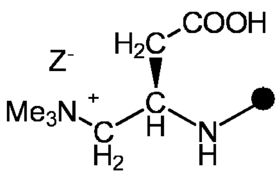
;
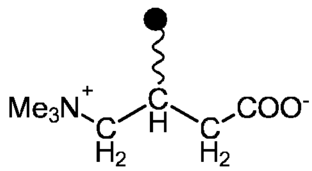
;

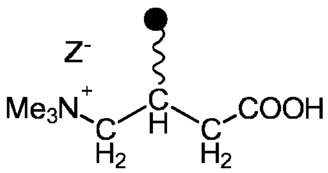
;
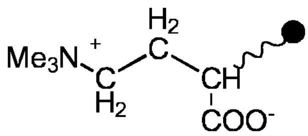
;
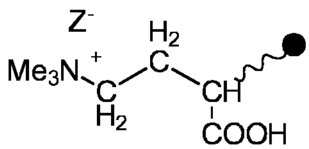

wherein a wavy bond indicates that the radical's carbon atom attached through it may be in either of the R and the S configuration

In one embodiment, R is selected from

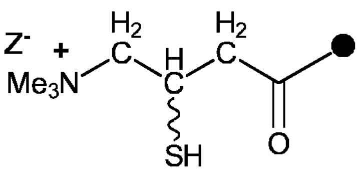
;
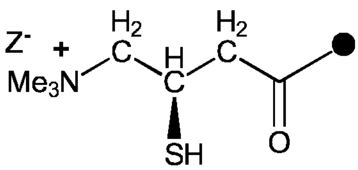
;

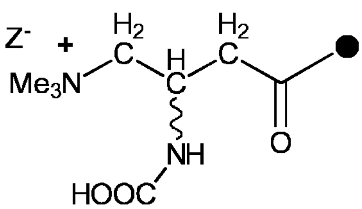
;
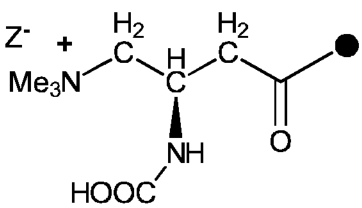
;

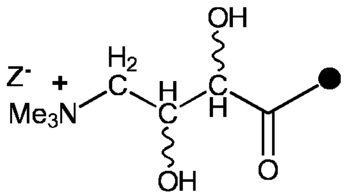

wherein a wavy bond indicates that the radical's carbon atom attached through it may be in either of the R and the S configuration.

"Bioactive molecule" means a compound having biological activity.

Bioactive molecules that can be incorporated according to the present invention include, without limitation, those selected from inorganic and organic drugs, nutraceutical and cosmeceutical drugs. Among the bioactive molecules one can, not limitedly, select those form the list of chemotherapeutic, anti-inflammatory, immunomodulatory, biocides, bactericides, antiviral agents, probiotics, nutraceuticals, enzymes, cytoprotectors, vaccines, polynucleotides, polypeptides, and polysaccharides.

In some embodiments, the bioactive molecule can be selected from indomethacin, curcumin, acyclovir, coumarin-6 and (R)-3-(3-tetradecylureido)-4-(trimethylammonium)butanoate.

The skilled person will be able to choose the appropriate pharmaceutically acceptable anion from those available to him. In one embodiment the pharmaceutically acceptable anion can be selected from hydroxyl, chloride, bromide, fluoride, iodide, phosphate, acetate, maleate, fumarate, lactate, tartrate, citrate, and gluconate.

In one embodiment Ra, Rb and Rc each are methyl.

The polymer is in the form of nanoparticles.

The synthetic processes implemented to produce nanoparticles allows to obtain nanoparticles of extremely small size and in any case not higher than 130 nm: such sizes imply a significantly advantageous cellular uptake compared to the nanoparticles with higher dimensions (Singh M, et al., Biotechnol Adv. 20: 5-6, 341-359, 2002).

In one embodiment, the nanoparticles have an average size below 160 nm.

In one embodiment, the nanoparticles have an average size of about 100 nm.

"Biocompatible" means capable of functioning or existing in contact with biological fluid and/or tissue of a living organism.

"Biodegradable" means able to be gradually chemically broken down or degraded by biological fluid and/or tissue of a living organism.

The skilled person will be able to choose the biocompatible and / or biodegradable polymer from the wide range at his disposal, depending on the desired properties. In some embodiments the biocompatible and / or biodegradable polymer is selected from polyhydroxyalkanoates, polyesters, and their copolymers.

In some embodiments the polyhydroxyalkanoate is selected from poly (3-hydroxypropionate), poly (3-hydroxybutyrate), poly (3-hydroxyvalerate), and their copolymers such as for example poly (3-hydroxybutyrate-co 3-hydroxyvalerate).

In some embodiments, the polyester can, for example, be selected from polycaprolactone, the polylactic acid such as poly-DL-lactide, polyglycolic acid, and their copolymers such as poly lactic-co-glycolic acid.

In one embodiment, the polymers of the invention can be used for transporting bioactive molecule inside cells that express an organic cationic transporter.

In one embodiment, such organic cationic transporter is selected from SLC22A4, SLC6A14 and SLC6A16

The skilled person will be able to choose the most suitable linker according to the particular polymer and the preparation process used. The linker may, for example, be one of the typical grafting agents that are used in the art. For example, and as shown here, if the polymer is a polyhydroxyalkanoate, the derivatization with radical A or B can take place on the polymer which has been previously grafted by reaction with glycidyl methacrylate.

The skilled person will easily recognize, on the basis of the scientific literature or the examples given here, how to carry out the synthesis of the polymer functionalized with R, A, B or C radicals, depending on the chosen polymer. By way of example, it may refer to the methodologies described in d'Ayala G.G., et al., J Biomed Mater Res A. 94: 619-30, 2010.

It is noteworthy that the functionalization process can take place directly on the neosynthesized nanoparticles, as already described (Calarco A., et al., The genotoxicity of PEIbased nanoparticles is reduced by acetylation of polyethylenimine amines in human primary cells.: 10-7, 2013).

The methods used for the encapsulation of bioactive molecules within functionalized polymeric vectors are known from the international scientific literature (e.g., Fathi M, and Barar J. Perspectives highlights on biodegradable polymeric nanosystems for targeted therapy of solid tumors.: 49-57, 2017).

Polymer nanoparticles according to this invention can be formulated within pharmaceutical compositions which

contain the ingredients (excipients, etc.) necessary or useful for their applicability in the pharmaceutical field. The skilled person will know, depending on the chosen route of administration and the particular condition to be treated, to choose these ingredients by referring to their knowledge and practices of the art.

The polymer nanoparticles according to this invention can be administered to patients by oral, rectal, dermal, intravenous, nasal or trans-mucosal. The forms of administration may be either solid or liquid.

The skilled person will also be able to determine the dosage of polymer nanoparticle to be administered, as well as the route and the form of administration, for example, based on the chosen bioactive molecule or the patient's needs.

## Examples

### Example 1 - Polymer derivatized with radical of formula C

Poly (3-hydroxybutyrate) was used for the synthesis of the polymer functionalized with (Z) -4- (trimethylammonium) but-2-enoate (CTB), to obtain a polymer derivatized both with both C1 and C2 radicals

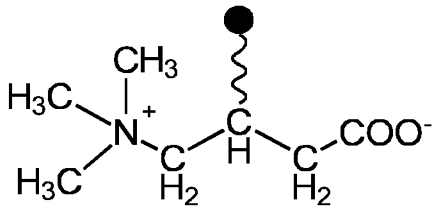

C1

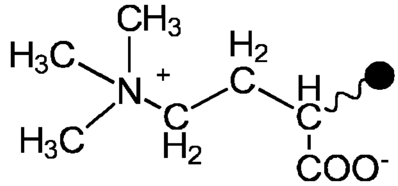

C2

In a standard reaction, 10g of poly (3-hydroxybutyrate) (PHB) was placed in a 250 mL flask, under magnetic stirring and nitrogen flow and left to dissolve in dimethylformamide (DMF, 100 mL) at 160° C. After complete dissolution of the polymer initiator, 0.2g (benzoyl peroxide) were added to the reaction mixture and the temperature brought to 80° C. (Z)-4-(trimethylammonium) but-2-enoate (CTB), in the form of an inner salt, was added to the reaction mixture and left under constant magnetic stirring, under a flow of nitrogen for a minimum of 4 hours. The molar ratio of polymer to functionalizing molecule is between 1: 5 and 1:20. At the end of the reaction, the product was repeatedly washed with bidistilled water and purified from residual monomers and homopolymers with warm methanol. The functionalized polymer was brought to constant weight under vacuum at 40 ° C.

PHB-CTB chloride was obtained by buffering the polymer derivatized with both C1 and C2 radicals with appropriate amounts of hydrochloric acid (mol / mol ratio from 1: 3 to 1: 5) at a pH between 5.3 and 5.9, to obtain a polymer derivatized with both C3 and C4 radicals.

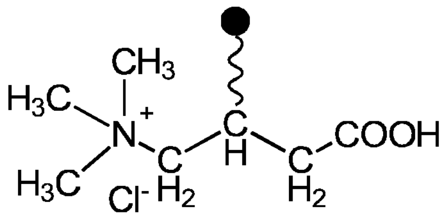

C3

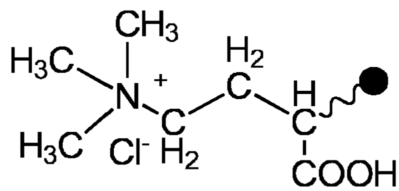

C4

The functionalization ratio (G%) was assessed using the formula
where $M_f$ is the polymer mass after functionalization, whereas e $M_i$ is the initial mass before functionalization.

$$G\% = \frac{M_f - M_i}{M_i} \times 100$$

The results are shown in Table 1 below

**Table 1**

| Derivatized polymer | Polymer/CTB Ratio (mol/mol) | Benzoyl peroxide (g) | DMF (ml) | Functionalization ratio (G%) |
|---|---|---|---|---|
| PHB-$CTB_5$ | 1:5 | 0.2 | 100 | 11% |
| PHB-$CTB_{10}$ | 1:10 | 0.2 | 100 | 18% |
| PHB-$CTB_{15}$ | 1:15 | 0.2 | 100 | 29% |
| PHB-$CTB_{20}$ | 1:20 | 0.2 | 100 | 43% |

These data show that the functionalization ratio increases with the CTB concentration and reaches a 43% value at a 1:20 polymer/CTB ratio.

NMR spectra were acquired on a Bruker Advance-600 MHz using $CDCl_3$ as solvent.

For PHB-$CTB_{20}$, the C1: C2 ratio was assessed by 13C NMR as being 30:70, as evidenced by the relative intensity of the 41 ppm and 41.9 ppm signals.

$^1$H NMR spectra confirmed the successful functionalisation, with expected signals at 2.8-3.2 ppm (9H, s, -N+$(CH_3)_3$) and 3.4-3.7 ppm (2H, m, -$NCH_2$-) and absence of signal between 6.1-7.2 ppm (2H, d, -HC=CH-).

This same procedure can be used for other hydroxyalkanoates such as poly (3-hydroxypropionate), poly (3-hydroxyvalerate), poly (3-hydroxybutyrate-co-3-hydroxyvalerate), and polyesters such as polycaprolactone, polylactic acid, polyglycolic acid and their copolymers.

**Example 2 - Polymer derivatized with radical of formula B**

In a standard reaction, 10 g of poly lactic-co-glycolic acid (PLGA) was reacted in a mixer at 80° C, with 2 g of glycidyl methacrylate (GMA) in the presence of 0.4 g of a radical initiator such as benzoyl peroxide and the reaction was carried out for 40 minutes. The modified polymer (PLGA-GMA) was extensively washed in methanol before being brought to constant weight.

In a subsequent step, 2 g of PLGA-GMA dissolved in 30 mL of chloroform were reacted in the presence of triethylamine with (3R)-3-Amino-4-(trimethylammonium)-butanoate (AMC) for 1 hr under magnetic stirring to obtain polymers derivatized with radicals B1.

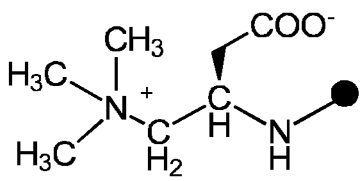

**B1**

As shown in Table 2 below, this reaction was carried out for polymer:AMC molar ratios comprised between 1:5 e 1:20. At the end of the reaction, the functionalized polymer (PLGA-AMC), was precipitated in methanol and brought to constant weight before being subjected to chemical-physical analysis, as detailed in Table 2 below.

The chloride of PLGA-AMC, represented by formula B2 below, was also obtained starting from B1 as described in example 1.

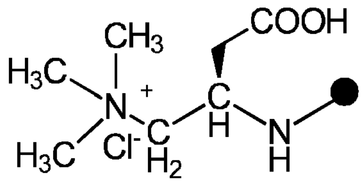

**B2**

**Table 2**

| Derivatized polymer | Polymer/AMC Ratio (mol/mol) | Benzoyl peroxide (g) | Glycidyl methacrylate (g) | Chloroform (mL) | Functionalization ratio (G%) |
|---|---|---|---|---|---|
| PLGA-$AMC_5$ | 1:5 | 0.4 | 2 | 30 | 12% |
| PLGA-$AMC_{10}$ | 1:10 | 0.4 | 2 | 30 | 16% |
| PLGA-$AMC_{15}$ | 1:15 | 0.4 | 2 | 30 | 25% |
| PLGA-$AMC_{20}$ | 1:20 | 0.4 | 2 | 30 | 39% |

These data show that the functionalization ratio increases with the AMC concentration and reaches a 39% value at a 1:20 polymer/CTB ratio.

$^1H$ NMR spectra confirmed the successful functionalisation, with expected signals at 2.8-3.2 ppm (9H, s, $-N+(CH_3)_3$) and 3.4-3.7 ppm (2H, m, $-NCH_2-$)

This same procedure can be used for other hydroxyalkanoates such as poly (3-hydroxypropionate), poly (3-hydroxyvalerate), poly (3-hydroxybutyrate-co-3-hydroxyvalerate), and polyesters such as polycaprolactone, polylactic acid, polyglycolic acid and their copolymers.

**Example 3** - **Polymer derivatized with radical of formula A**

Starting from commercially available D,L-thiocarnitine (TIC) and D,L-carboxyaminocarnitine (CAC) and dihydroxycarnitine (DIC) as internal salts and with the procedure described in example 2, PHB and PLGA polymers derivatized with radicals A1, A2 and A3 were obtained.

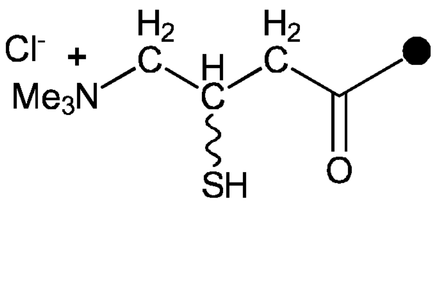

A1

;

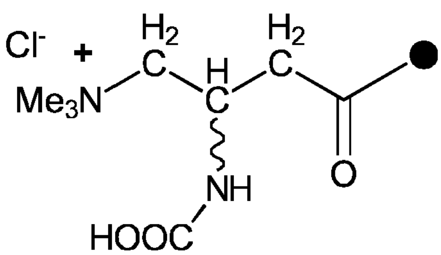

A2

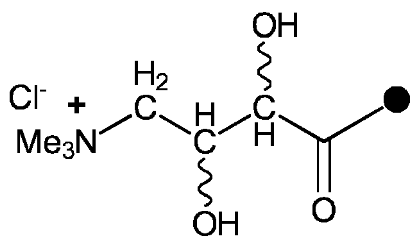

A3

Their characteristics are detailed in Tables 3-5 below

**Table 3**

| Derivatized polymer | Polymer/TIC Ratio (mol/mol) | Benzoyl peroxide (g) | Glycidyl methacrylate (g) | Chloroform (mL) | Functionalization ratio (G%) |
|---|---|---|---|---|---|
| PHB-$TIC_5$ | 1:5 | 0.4 | 2 | 35 | 13% |
| PHB-$TIC_{10}$ | 1:10 | 0.4 | 2 | 35 | 19% |

(continued)

| Derivatized polymer | Polymer/TIC Ratio (mol/mol) | Benzoyl peroxide (g) | Glycidyl methacrylate (g) | Chloroform (mL) | Functionalization ratio (G%) |
|---|---|---|---|---|---|
| PHB-$TIC_{15}$ | 1:15 | 0.4 | 2 | 35 | 28% |
| PHB-$TIC_{20}$ | 1:20 | 0.4 | 2 | 35 | 43% |

**Table 4**

| Derivatized polymer | Polymer/CAC Ratio (mol/mol) | Benzoyl peroxide (g) | Glycidyl methacrylate (g) | Chloroform (mL) | Functionalization ratio (G%) |
|---|---|---|---|---|---|
| PLGA-$CAC_5$ | 1:5 | 0.4 | 2 | 27 | 11% |
| PLGA-$CAC_{10}$ | 1:10 | 0.4 | 2 | 27 | 16% |
| PLGA-$CAC_{15}$ | 1:15 | 0.4 | 2 | 27 | 31% |
| PLGA-$CAC_{20}$ | 1:20 | 0.4 | 2 | 27 | 33% |

**Table 5**

| Derivatized polymer | Polymer/DIC Ratio (mol/mol) | Benzoyl peroxide (g) | Glycidyl methacrylate (g) | Chloroform (mL) | Functionalization ratio (G%) |
|---|---|---|---|---|---|
| PHB-$DIC_5$ | 1:5 | 0.4 | 2 | 30 | 10% |
| PHB-$DIC_{10}$ | 1:10 | 0.4 | 2 | 30 | 14% |
| PHB-$DIC_{15}$ | 1:15 | 0.4 | 2 | 30 | 19% |
| PHB-$DIC_{20}$ | 1:20 | 0.4 | 2 | 30 | 21% |

**Example 4** - **Nanoparticles preparation**

Nanoparticles were prepared by nanoprecipitation, solubilizing the functionalized polymer (PHB-CTB20, PLGA-AMC20, PHB-TIC20, PLGA-CAC20 and PHB-DIC20) and the bioactive molecules to be encapsulated such as curcumin (CUR), indomethacin (IND), acyclovir (ACY), Coumarin-6 or (R)-3-(3-tetradecylureido)-4-(trimethylammonium)butanoate (TB), in a solvent compatible both with the type of molecule to be encapsulated and with the selected polymer (e.g. dimethylformamide) under magnetic stirring at a temperature of 50 ° C. After complete solubilization, the mixture was dripped into aqueous solution of Pluronic F127 (between 0.1 to 0.5% w / v) and left under stirring until complete precipitation of the loaded nanoparticulate. Alternative techniques for the formation of nanoparticulate, such as the oil / water emulsion (o / w), have yielded comparable results. The nanoparticles were separated by centrifugation (15,000 rpm, 45 min) and washed extensively with milliQ water. The size and surface charge of the nanoparticles was evaluated using a dynamic light scattering system (Malven, UK).

Tables 6-12, where each value is the average of three measurements made, show the data on the chemical-physical characterization of drug loaded nanoparticles obtained in examples 1 to 3.

**Table 6**

| Drug | Derivatized Polymer | Drug/Polymer ratio | Size [nm] | Polydiversity index | Zeta potential [mV] | Encapsulation efficiency [%] |
|---|---|---|---|---|---|---|
| CUR | PHB-$CTB_{20}$ | 5:1 | 94.3 ± 2.6 | 0.074 ± 0.004 | +15.3 ± 1.5 | 78.3 ± 2.8 |
| | PHB-$CTB_{20}$ | 10:1 | 99.1 ± 3.1 | 0.081 ± 0.009 | +16.2 ± 2.9 | 84.1 ± 2.6 |
| | PHB-$CTB_{20}$ | 20:1 | 112.6 ± 4.6 | 0.091 ± 0.013 | +16.4 ± 2.6 | 89.1 ± 3.8 |

(continued)

| Drug | Derivatized Polymer | Drug/Polymer ratio | Size [nm] | Polydiversity index | Zeta potential [mV] | Encapsulation efficiency [%] |
|---|---|---|---|---|---|---|
| IND | PHB-CTB$_{20}$ | 5:1 | 96.3 ± 4.5 | 0.064 ± 0.006 | +15.5 ± 1.5 | 83.2 ± 3.8 |
| | PHB-CTB$_{20}$ | 10:1 | 103.2 ± 3.1 | 0.073 ± 0.008 | +15.9 ± 2.6 | 89.2 ± 5.3 |
| | PHB-CTB$_{20}$ | 20:1 | 113.5 ± 7.3 | 0.098 ± 0.016 | +16.9 ± 3.6 | 91.1 ± 4.6 |
| ACY | PHB-CTB$_{20}$ | 5:1 | 89.6 ± 2.8 | 0.085 ± 0.011 | +15.9 ± 1.9 | 75.3 ± 2.9 |
| | PHB-CTB$_{20}$ | 10:1 | 97.5 ± 4.3 | 0.093 ± 0.012 | +16.2 ± 2.7 | 77.5 ± 6.3 |
| | PHB-CTB$_{20}$ | 20:1 | 106.4±3.5 | 0.113 ± 0.016 | +17.2 ± 3.7 | 81.6 ± 4.3 |

**Table 7**

| Drug | Derivatized Polymer | Drug/Polymer ratio | Size [nm] | Polydiversity index | Zeta potential [mV] | Encapsulation efficiency [%] |
|---|---|---|---|---|---|---|
| CUR | PLGA-AMC$_{20}$ | 5:1 | 96.4 ± 1.8 | 0.056 ± 0.004 | +18.5 ± 0.5 | 85.2 ± 6.5 |
| | PLGA-AMC$_{20}$ | 10:1 | 99.5± 1.1 | 0.069 ± 0.002 | +18.9 ± 0.9 | 89.3 ± 3.6 |
| | PLGA-AMC$_{20}$ | 20:1 | 108.6 ± 4.3 | 0.086 ± 0.012 | +18.2 ± 0.6 | 71.3 ± 4.7 |
| IND | PLGA-AMC$_{20}$ | 5:1 | 95.3 ± 2.7 | 0.048 ± 0.007 | +19.8 ± 1.2 | 81.4 ± 5.4 |
| | PLGA-AMC$_{20}$ | 10:1 | 103.3 ± 4.2 | 0.071 ± 0.006 | +17.9 ± 0.4 | 93.1 ± 6.1 |
| | PLGA-AMC$_{20}$ | 20:1 | 111.3 ± 5.9 | 0.094 ± 0.007 | +18.9 ± 1.1 | 71.3 ± 5.6 |
| ACY | PLGA-AMC$_{20}$ | 5:1 | 91.4 ± 3.2 | 0.086 ± 0.008 | +19.9 ± 1.3 | 78.2 ± 3.3 |
| | PLGA-AMC$_{20}$ | 10:1 | 96.1 ± 2.4 | 0.097 ± 0.008 | +18.8 ± 1.7 | 88.1 ± 5.2 |
| | PLGA-AMC$_{20}$ | 20:1 | 107.4 ± 5.3 | 0.106 ± 0.018 | +18.4 ± 0.6 | 70.6 ± 4.3 |

**Table 8**

| Drug | Derivatized Polymer | Drug/ Polymer ratio | Size [nm] | Polydiversity index | Zeta potential [mV] | Encapsulation efficiency [%] |
|---|---|---|---|---|---|---|
| CUR | PHB-$TIC_{20}$ | 5:1 | 99.1 ± 2.6 | 0.076 ± 0.008 | +14.6 ± 2.5 | 69.3 ± 3.7 |
| | PHB-$TIC_{20}$ | 10:1 | 103.4 ± 3.1 | 0.091 ± 0.011 | +15.7 ± 1.8 | 73.1 ± 2.7 |
| | PHB-$TIC_{20}$ | 20:1 | 98.8 ± 4.6 | 0.075 ± 0.009 | +14.3 ± 2.4 | 71.1 ± 4.8 |
| IND | PHB-$TIC_{20}$ | 5:1 | 102.5 ± 4.5 | 0.078 ± 0.007 | +18.5 ± 1.5 | 75.2 ± 4.2 |
| | PHB-$TIC_{20}$ | 10:1 | 106.7± 3.1 | 0.094 ± 0.013 | +17.9 ± 2.6 | 79.2 ± 5.4 |
| | PHB-$TIC_{20}$ | 20:1 | 104.5 ± 7.3 | 0.095 ± 0.012 | +14.9 ± 3.6 | 77.1 ± 4.6 |
| ACY | PHB-$TIC_{20}$ | 5:1 | 92.5 ± 2.8 | 0.097 ± 0.014 | +16.9 ± 1.9 | 79.3 ± 4.9 |
| | PHB-$TIC_{20}$ | 10:1 | 95.6 ± 4.3 | 0.091 ± 0.014 | +14.7 ± 2.7 | 81.5 ± 6.3 |
| | PHB-$TIC_{20}$ | 20:1 | 107.2 ±3.5 | 0.099 ± 0.011 | +18.1 ± 4.7 | 80.6 ± 3.3 |

**Table 9**

| Drug | Derivatized Polymer | Drug/ Polymer ratio | Size [nm] | Polydiversity index | Zeta potential [mV] | Encapsulation efficiency [%] |
|---|---|---|---|---|---|---|
| CUR | PLGA-$CAC_{20}$ | 5:1 | 99.3 ± 1.8 | 0.059 ± 0.004 | +17.5 ± 0.5 | 79.2 ± 4.5 |
| | PLGA-$CAC_{20}$ | 10:1 | 97.2± 1.1 | 0.071 ± 0.002 | +17.9 ± 0.9 | 76.3 ± 2.8 |
| | PLGA-$CAC_{20}$ | 20:1 | 98.1 ± 4.3 | 0.087± 0.012 | +19.2 ± 0.6 | 73.3 ± 4.4 |
| IND | PLGA-$CAC_{20}$ | 5:1 | 102.3 ± 2.7 | 0.054 ± 0.007 | +17.8 ± 1.2 | 84.2 ± 5.4 |
| | PLGA-$CAC_{20}$ | 10:1 | 105.3 ± 3.2 | 0.056 ± 0.006 | +18.3 ± 0.4 | 88.4 ± 6.1 |
| | PLGA-$CAC_{20}$ | 20:1 | 113.3 ± 6.1 | 0.063 ± 0.007 | +19.6± 1.1 | 84.1 ± 5.6 |
| ACY | PLGA-$CAC_{20}$ | 5:1 | 97.2 ± 3.9 | 0.081 ± 0.008 | +18.2 ± 1.3 | 81.2 ± 3.3 |
| | PLGA-$CAC_{20}$ | 10:1 | 99.4 ± 5.4 | 0.072 ± 0.008 | +19.4 ± 1.7 | 86.6 ± 5.2 |
| | PLGA-$CAC_{20}$ | 20:1 | 106.8 ± 7.3 | 0.83 ± 0.018 | +17.2 ± 0.6 | 75.3 ± 4.3 |

**Table 10**

| Drug | Derivatized Polymer | Drug/Polymer ratio | Size [nm] | Polydiversity index | Zeta potential [mV] | Encapsulation efficiency [%] |
|---|---|---|---|---|---|---|
| CUR | PLGA-$DIC_{20}$ | 5:1 | 95.4 ± 2.5 | 0.076 ± 0.011 | ±18.6 ± 1.4 | 69.3 ± 3.8 |
| | PLGA-$DIC_{20}$ | 10:1 | 98.1± 4.1 | 0.108 ± 0.011 | +19.1 ± 3.9 | 66.4 ± 2.9 |
| | PLGA-$DIC_{20}$ | 20:1 | 102.3 ± 3.2 | 0.088 ± 0.009 | +18.3 ± 1.9 | 65.4 ± 2.9 |
| IND | PLGA-$DIC_{20}$ | 5:1 | 97.7 ± 3.7 | 0.068 ± 0.009 | +17.4 ± 3.2 | 74.1 ± 3.4 |
| | PLGA-$DIC_{20}$ | 10:1 | 106.4 ± 3.2 | 0.081 ± 0.008 | +19.1 ± 2.1 | 76.5 ± 4.1 |
| | PLGA-$DIC_{20}$ | 20:1 | 104.6 ± 4.9 | 0.105 ± 0.015 | +18.4 ± 4.1 | 72.8 ± 4.1 |
| ACY | PLGA-$DIC_{20}$ | 5:1 | 106.3 ± 2.9 | 0.096 ± 0.012 | +18.6 ± 3.3 | 76.1 ± 2.4 |
| | PLGA-$DIC_{20}$ | 10:1 | 104.9 ± 2.7 | 0.094 ± 0.009 | +17.8 ± 2.7 | 78.5 ± 3.3 |
| | PLGA-$DIC_{20}$ | 20:1 | 99.4 ± 3.3 | 0.096 ± 0.014 | +19.2 ± 1.3 | 79.1 ± 2.6 |

**Table 11**

| Drug | Derivatized Polymer | Drug/ Polymer ratio | Size [nm] | Polydiversity index | Zeta potential [mV] | Encapsulation efficiency [%] |
|---|---|---|---|---|---|---|
| Coumarin-6 | PHB-TIC$_{20}$ | 5:1 | 86.3 ± 1.9 | 0.079 ± 0.009 | +16.3 ± 1.4 | 69.3 ± 3.4 |
| | PHB-TIC$_{20}$ | 10:1 | 82.3 ± 2.5 | 0.081 ± 0.012 | +16.8 ± 2.5 | 76.4 ± 6.3 |
| | PHB-TIC$_{20}$ | 20:1 | 89.2 ±1.6 | 0.094 ± 0.016 | +16.8 ± 2.8 | 73.2 ± 4.3 |
| | PHB-CTB$_{20}$ | 5:1 | 84.3 ± 2.3 | 0.081 ± 0.011 | +15.7 ± 2.4 | 69.8 ± 5.2 |
| | PHB-CTB$_{20}$ | 10:1 | 89.2 ± 4.3 | 0.093 ± 0.012 | +16.3 ± 1.7 | 71.3 ± 2.3 |
| | PHB-CTB$_{20}$ | 20:1 | 93.2 ±2.5 | 0.096 ± 0.009 | +16.2 ± 2.7 | 68.1 ± 2.8 |
| | PHB-DIC$_{20}$ | 5:1 | 83.4 ± 2.4 | 0.085 ± 0.011 | +16.4 ± 2.4 | 71.3 ± 3.5 |
| | PHB-DIC$_{20}$ | 10:1 | 86.5 ± 2.3 | 0.088 ± 0.008 | +16.8 ± 1.7 | 75.2 ± 6.3 |
| | PHB-DIC$_{20}$ | 20:1 | 95.7 ±3.5 | 0.094 ± 0.011 | +17.4 ± 1.9 | 70.1 ± 4.3 |
| | PLGA-AMC$_{20}$ | 5:1 | 87.6 ± 1.8 | 0.082 ± 0.008 | +15.7 ± 2.9 | 59.4 ± 1.8 |
| | PLGA-AMC$_{20}$ | 10:1 | 94.5 ± 2.3 | 0.088 ± 0.011 | +16.3 ± 3.1 | 77.5 ± 3.6 |
| | PLGA-AMC$_{20}$ | 20:1 | 98.4 ± 2.1 | 0.113 ± 0.014 | +17.6 ± 2.4 | 70.2 ± 1.8 |
| | PLGA-CAC$_{20}$ | 5:1 | 85.1 ± 1.4 | 0.091 ± 0.006 | +16.8 ± 2.5 | 65.2 ± 3.5 |
| | PLGA-CAC$_{20}$ | 10:1 | 89.5 ± 1.3 | 0.096 ± 0.010 | +16.9 ± 2.7 | 68.3 ± 3.8 |
| | PLGA-CAC$_{20}$ | 20:1 | 94.6 ± 2.5 | 0.099 ± 0.011 | +17.8 ± 3.7 | 61.2 ± 4.3 |

**Table 12**

| Drug | Derivatized Polymer | Drug/ Polymer ratio | Size [nm] | Polydiversity index | Zeta potential [mV] | Encapsulation efficiency [%] |
|---|---|---|---|---|---|---|
| TB | PLGA-AMC$_{20}$ | 5:1 | 103.6 ± 2.9 | 0.098 ± .008 | +22.5 ± 3.5 | 76.4 ± 2.5 |
| | PLGA-AMC$_{20}$ | 10:1 | 108.4± 3.6 | 0.096 ± .010 | +24.3 ± 1.6 | 78.5 ± 4.16 |
| | PLGA-AMC$_{20}$ | 20:1 | 112.5 ± 2.3 | 0.103± 0.009 | +25.1 ± 3.4 | 74.3 ± 2.7 |

These analyses show that a concentration of 0.5% Pluronic and a 12-hour precipitation time allows to obtain an encapsulation rate higher than 65% for all the bioactive molecules considered with an excellent yield of nanoparticulate. The size of the nanoparticles produced are between 87.1 ± 3.8 and 113.5 ± 7.3, which significantly facilitates transcellular transport. In fact, it has been demonstrated that nanoparticles with a diameter of around 100 nm are able to reach the submucosal area of the intestine in vivo, while the particles with a diameter greater than 140-160 nm remain confined mainly in the surface epithelium.

The potential ζ increases proportionally to the charge density due to the functionalization and is between +1.9 ± 0.9 and +19.8 ± 1.2. The data obtained with the potential ζ were confirmed by X-ray photoelectron spectroscopy (XPS) analysis in which the amount of nitrogen (index of the presence of the trimethyl ammonium group) increases proportionally with the increase in the degree of functionalization. The stability of the nanoparticles produced was determined in suitable liquids such as phosphate buffer, human plasma, simulated intestinal fluid and simulated gastric liquid. In all the liquids analyzed, the dimensions of the nanoparticles produced did not show significant variations even after 1 month.

**Example 5** - **Biocompatibility**

The in vitro biocompatibility of functionalized nanodevices of the invention was analyzed by direct and indirect cytotoxicity assays in accordance with current legislation (IS EN ISO 10993-5: Biological evaluation of medical devices - Part 5: Tests for in vitro cytotoxicity (2009)), using both cell lines (kidney embryonic cells, HEK293 and epithelial cells of the conjunctiva HCjE) and primary cells (human primary fibroblasts, HF) kept in culture according to the conditions provided by the manufacturer.

**MTT Test**

The MTT (dimethyl-thiazolyl-diphenyl-tetrazolium bromide) test (MTT Cell Proliferation Assay Kit, Invitrogen) is a quantitative colorimetric assay. The assay is based on the ability of the mitochondrial succinate dehydrogenase enzymes present in the viable cells to transform the MTT salt tetrazolium 3- (4,5-dimetiltiazole-2-yl) -2,5-diphenyltetrazolium bromide, yellow, in crystals of formazan, by tetrazole ring rupture. The crystals have a purple color and are insoluble in aqueous solutions. The crystals redissolved in acidified isopropanol form a purple solution measured spectrophotometrically at 570 nm. The absorbance values are proportional to the amount of formazan produced and, therefore, to the metabolic activity and cell viability.

This test provides a relative indication of the vital population and allows to evaluate the toxicity of a substance, by comparing the cell viability indices obtained from the treated cells with respect to the controls.

The cells were seeded in 96-well plates at a density of between $1.5\times10^3$ and $2.0\times10^3$ cells / well in a suitable culture medium devoid of phenol red. After 24 hours incubation at 37 ° C, the cells were treated with different concentrations of nanodevices (5, 50, 100, 250 and 500 $\mu$g / ml) in complete medium without phenol red. Each sample was tested in triplicate and the tests were repeated three times. Untreated cells were used as a negative control. After 24, 48 and 72 hours of treatment, the medium was aspirated, and the cells were incubated with 150 $\mu$g / well of a 1 mg / ml MTT solution, for 3 hours at 37 ° C / 5% $CO_2$. The solution was removed and replaced with 200 $\mu$l / well of acidified isopropanol and left under magnetic stirring at room temperature for 30 minutes. Spectrophotometric analysis was performed at 570 nm using a microplate reader (Cytation3, ASHI). Figures 1 and 2 show the results obtained expressed as cell viability (%) calculated according to the formula:

$$\%\ \text{cell viability} = [OD_{570nm}\ \text{tested product} / OD_{570nm}\ \text{negative control}] \times 100$$

for PHB-$CTB_{20}$ of example 1 and PLGA-$AMC_{20}$, of example 2, respectively.

As shown in Figures 1 and 2, the synthesized nanodevices did not induce, at the concentrations tested, a reduction in cell viability above the threshold value of 80% compared to the control (cells grown in the absence of nanoparticulates) as reported by the Annex C of the ISO 10093-5 standard for which it is possible to define the non-cytotoxicity of the same.

**Example 6A** - **Internalization experiments**

The ability of the nanodevices to be internalized by the cells was evaluated by HPLC on HEK293 that overexpress SLC22A4 (HEK293-A4), SLC6A14 (HEK293-A14) and SLC22A16 (HEK293-A16) after stable transfection with appropriate cDNA coding for proteins of interest. The cells were seeded in 24-well plates at a density of $1.5\times10^5$ cells / well in appropriate culture medium devoid of phenol red. After 24 hours incubation at 37°C the cells were incubated or not with anti-SLC22A4 antibodies, anti-SLC22A14, anti-SLC22A16. After 3 hours, drug loaded nanoparticles of example 4 were added and after either 1,3 and 8 hours or 5,10,15,20 and 40 minutes, the cells were thoroughly washed with phosphate buffer, lysed and the intracellular content of drug was determined by HPLC. The results are shown in Figs 3 to 9, as detailed in Table 13 below.

**Table 13**

| Figure | Nanoparticle | Drugs | Drug/ polymer ratio | Loaded Nanoparticle concentration | Incubation times |
|---|---|---|---|---|---|
| Fig.3 | PHB-$CTB_{20}$ | Curcumin, Indomethacin, Acyclovir | 1:20 | 100μg/ml | 1.3 and 8 hours |
| Fig. 4 | PLGA-$AMC_{20}$ | Curcumin, Indomethacin, Acyclovir | 1:10 | 100μg/ml | 1.3 and 8 hours |
| Fig. 5 | PLGA-$AMC_{20}$ | Coumarin-6 | 1:20 | 10 μg/ml | 0.5,10.15, 20 and 40 minutes |
| Fig. 6 | PHB-$CTB_{20}$ | Coumarin-6 | 1:20 | 10 μg/ml | 0.5,10.15, 20 and 40 minutes |
| Fig.7 | PLGA-$TIC_{20}$ | Coumarin-6 | 1:20 | 10 μg/ml | 0.5,10.15, 20 and 40 minutes |
| Fig. 8 | PHB-$CAC_{20}$ | Coumarin-6 | 1:20 | 10 μg/ml | 0.5,10.15, 20 and 40 minutes |
| Fig.9 | PLGA-$DIC_{20}$ | Coumarin-6 | 1:20 | 10 μg/ml | 0.5,10.15, 20 and 40 minutes |

All nanoparticles used in the above table have chloride as a counterion.

These data show that the polymers of the invention can efficiently deliver drugs due to their interaction with SLC22A4, SLC6A14, and SLC22A16.

**Example 6B** - **Comparative example with betaine**

As a comparative example with betaine of the prior art, internalization data were also acquired as described in example 6A on drug loaded PLGA nanocapsules named PLGA-$CTI_{20}$, derivatized with the following groups

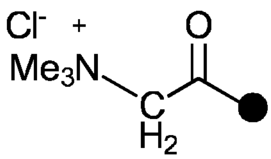

And obtained as described in examples 2 and 4 from 1-carbossi-N,N,N-trimetil-, hydroxide (CTI), and which characteristics are listed in Table 14 below

**Table 14**

| Drug | Derivatized Polymer | Drug/ Polymer ratio | Size [nm] | Polydiversity index | Zeta potential [mV] | Encapsulation efficiency [%] |
|---|---|---|---|---|---|---|
| CUR | PLGA-$CTI_{20}$ | 10:1 | 98.9:1: 4.1 | 0.108 ±0.011 | +17.9 ± 3.9 | 81.2 ± 2.9 |
| IND | PLGA-$CTI_{20}$ | 10:1 | 98.4 ± 3.2 | 0.081 ±0.008 | +16.9 ± 2.1 | 85.6 ± 4.1 |
| ACY | PLGA-$CTI_{20}$ | 10:1 | 98.4 ± 2.7 | 0.094 ±0.009 | +16.8 ± 2.7 | 84.1 ± 3.3 |
| Coumarin-6 | PLGA-$CTI_{20}$ | 20:1 | 95.4 ± 2.1 | 0.106 ±0.014 | +18.1 ± 3.4 | 66.4 ± 3.1 |

The results of such internalization experiments on drug-loaded PLGA-$CTI_{20}$ nanoparticles are shown in Figures

10 and 11 as detailed in table 15 and show by comparison to Figures 3-9 that, even though at 1,3 and 8 hours there is no significant difference in the internalization efficiency between a betaine-derived polymer and those of the invention, there is a surprising superiority at much shorter (up to 40 minutes) times.

**Table 15**

| **Figure** | **Nanoparticle** | **Drugs** | **Drug/ polymer ratio** | **Loaded Nanoparticle concentration** | **Incubation times** |
|---|---|---|---|---|---|
| Fig. 10 | PLGA-$CTI_{20}$ | Curcumin, Indomethacin, Acyclovir | 1:10 | 100μg/ml | 1,3 and 8 hours |
| Fig. 11 | PLGA-$CTI_{20}$ | Coumarin-6 | 1:20 | 10 μg/ml | 0,5,10,15, 20 and 40 minutes |

**Example 6C** - **Comparative example with carnitine**

As a comparative example with the carnitine of the prior art, internalization data were also acquired as described in example 6A with 10 μg/ml drug loaded PLGA nanoparticles named PLGA-$CAR_{20}$ derivatized with the following group:

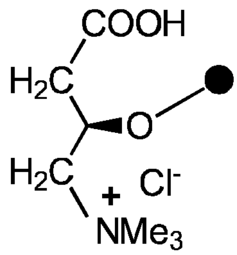

,

obtained as described in examples 2 and 4 starting from L-carnitine (CAR) internal salt, and which characteristics are listed in Table 16 below

**Table 16**

| Drug | Derivatized Polymer | Drug/ Polymer ratio | Size [nm] | Polydiversity index | Zeta potential [mV] | Encapsulation efficiency [%] |
|---|---|---|---|---|---|---|
| Coumarin-6 | PLGA-$CTI_{20}$ | 20:1 | 98.1± 4.5 | 0094 ± 0.09 | +16.9 ± 3.6 | 68.4.2 ± 2.3 |

The results are shown in Fig. 12, and by comparison with those of Figures 5 to 9, show how the polymers of the invention much better perform in terms of rapid delivery.

**Example 7- Specificity**

To verify the specificity of the interaction of the nanodevices of the invention towards the SLC22A4, SLC6A14 and SLC22A16 transporters the experiment described in example 6A was carried out on PHB nanoparticles loaded with coumarin-6, in which the positive charge is obtained either by lysine functionalization (PHB-Lys) in which the lysine is bound to the polymer through the epsilon NH2 group or by functionalisation with chitosan (PHB-Chito), in which the chitosan is bound to the polymer by the primary alcohol group.

HEK293 which permanently hyper-express SLC22A4 (HEK293-A4), SLC6A14 (HEK293-A14) and SLC22A16 (HEK293-A16) were seeded in 24-well plates at a density of $1.5\times10^5$ cells / well in appropriate culture medium of phenol red. After 24 hours incubation at 37 ° C the cells were incubated or not with anti-SLC22A4 antibodies, anti-SLC22A14, anti-SLC22A16. After 3 hours, 100μg / ml of PHB-$CTB_{20}$, of example 4 PHB-Lys or PHB-Chito were added and each sample was tested in triplicate and the tests were repeated three times. Untreated cells were used as a negative control. After 1, 3 and 8 hours of treatment, the cells were extensively washed with phosphate buffer, lysed and the intracellular

content of coumarin-6 quantized by HPLC as reported by Calarco et al. [Toxicology Letters. 2013. 218 (1): 10-17].

The results of this experiment, shown in Figure 13, demonstrate that:

a. even after 8 hours of incubation with PHB-Lys and PHB-Chito, the amount of coumarin-6 present inside the cells is significantly lower than that obtained with PHB-$CTB_{20}$;
b. the internalization kinetics of PHB-Lys and PHB-Chito are not influenced by the presence of specific antibodies to the various OCTs unlike what happens with PHB-$CTB_{20}$;
c. the uptake of PHB-$CTB_{20}$ is significantly faster than PHB-Lys and PHB-Chito

**Example 8- Curcumin Bioavailability**

Animal experiments were carried out at the University of Naples, "Federico II", after approval of the experimentation by the University Ethics Committee and the Ministry of Health. Sprague Dawley rats weighing about 160-180 grams were used for the experimentation. Animals had free access to water and food and were kept in a 12-hour light / dark ratio. Before the trial began, animals were acclimatized for a week and fed ad libitum on a commercial diet. The animals, divided into three groups (Table 17), were treated directly in the gastric lumen by inserting a gastric probe via the oro-esophageal route. Blood samples from the retroorbital arteries were collected at predetermined times, under anaesthesia, and stored at -70 ° C until further analysis.

**Table 17**

| Group | Number of animals | | Dose | Blood draw |
|---|---|---|---|---|
| A | 8 | Free curcumin | 50mg/kg | At 0, 24, 48 hours |
| B | 10 | Curcumin-loaded PHB-CTB nanoparticles of the invention | 50mg/kg | At 0, 2, 4, 6 and 8 hours |
| C | 10 | Curcumin-loaded PHB-CTB nanoparticles of the invention | 50mg/kg | At 0, 12, 24, and 48 hours |

Plasma samples were submitted to an extraction process. Each sample was acidified with 10 μl of 6M HCl, then treated with 10U of β-glucuronidase and incubated at 37 ° C for 45 minutes. The extraction was then carried out with 2 volumes of Methanol / Chloroform (1: 2 v / v). All the extracts thus obtained were concentrated in Savant apparatus and then analyzed using the previously described HPLC method.

Figure 13 shows the results concerning the in vivo bioavailability of curcumin encapsulated after oral administration compared to free curcumin.

The results obtained showed that after oral administration the systemic absorption of free curcumin is at the limit of the sensitivity of the method, while for the curcumin encapsulated in PHB-CTB nanoparticles of the invention, the peak of absorption is obtained at 6 hours with a concentration equal to 760 ng / ml. The (PHB-CTB) derivatized polymer used in this example has as counter-ion Z of the formula X the chloride ion.

**Example 9- TB Systemic exposure**

(R)-3-(3-tetradecylureido)-4-(trimethylammonium)butanoate (TB)-loaded PLGA-$AMC_{20}$ nanoparticles of example 4 were used in an in vivo experiment to assess systemic exposure to the drug.

7 weeks old Sprague Dawley rats weighing about 280-310g were used. Rats were divided into two groups of 8 animals each and kept in cages under controlled atmospheric conditions of 22 ± 2 ° C temperature, 55 ± 15% relative humidity, about 15-20 filtered air changes / hour and a circadian rhythm of 12 hours of artificial light per day (7 am to 7pm). Animal were kept in groups of 4/cage before treatment and in individual cages during treatment, and had free access to food and water at all times.

The day before the study, animals were implanted with a permanent blood collection catheter under anesthesia.

On the day of the study, the first group was administered orally with 100 mg/kg of a 10 mg/mL water suspension of TB, whereas the second group was administered orally, in a single dose, of a water suspension of TB-loaded PLGA-$AMC_{20}$ nanoparticles of the invention in such quantity so as to administer 100 mg TB/kg. The water suspensions used were equivalent to 10 mg TB/ml.

100 mL blood draws were taken at before administration and at 0.5, 1, 2, 2.5, 3, 4, 6, 8, 10 and 24 hours after administration in heparinized test tubes, centrifuged at 3000 rpm for 10 minutes. Plasma was then transferred to Eppendorf

tubes and frozen (-20°C) until analysis.

TB concentration within these plasma samples were determined by HPLC/MS/MS by derivation from calibration curves. Accuracy and precision were derived from the analysis of quality control samples. Interassay accuracy and precision were less than 6.6%.

The results of this experiment are shown in Figure 15, and clearly show an improved systemic exposure to the drug when formulated with the nanoparticles of the invention.

**Claims**

**1.** A bioactive molecule delivery system comprising:

a) a bioactive molecule; and
b) a biocompatible and/or biodegradable polymer in the form of nanoparticles covalently derivatized with a radical of formula R

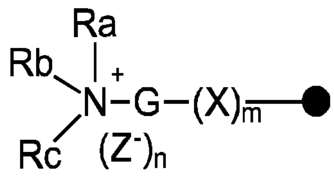

**R**

,

optionally via linker, and wherein:

● is the point of attachment to the polymer, or, when a linker is present, to the linker;
Ra, Rb and Rc can be the same or different and are selected from methyl and ethyl;
G is a $C_1$-$C_5$ linear, branched or cyclic alkyl, optionally substituted with one or more groups selected from hydroxy, carboxyl, carboxylate, 1-pyrrolidinyl, 1-azetidinyl, 1-aziridinyl, sulfhydryl, -NRdRe and -N=NRf;
Rd, Re and Rf are independently selected from hydrogen, methyl, ethyl and propyl;
m can be zero or 1;
-X- is selected form the list of -C(O)-, -NRg-, -S- and -$SO_2$-,
Rg is selected from hydrogen, methyl, ethyl and propyl;
Z is a pharmaceutically acceptable anion;
n can be zero or 1, and it can only be zero when G bears a carboxylate;
with the proviso that R cannot be

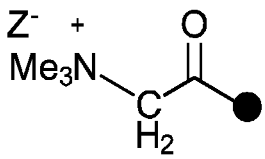

**2.** System according to claim 1, wherein the radical R is selected from radicals of formula A, B and C

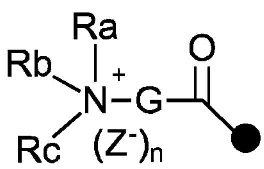

**A**

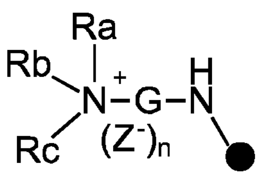

**B**

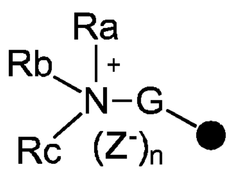

**C**

where ● , Ra, Rib, Rc, G, Z and n are as defined in claim 1.

**3.** System according to claim 2, wherein the radical R is selected from

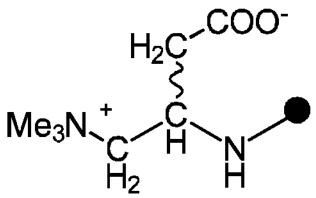 ; 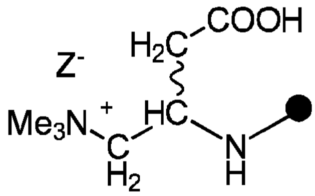 ;

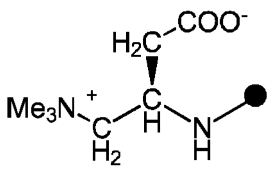 ; 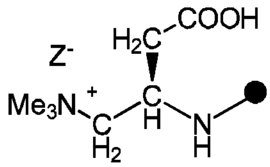 ; 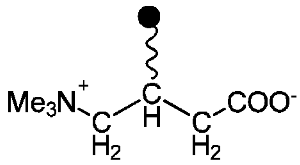 ;

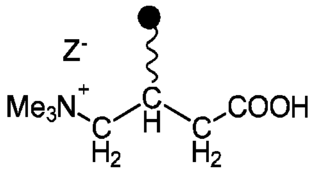 ; 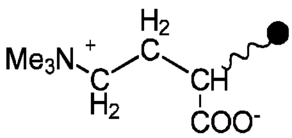 ; 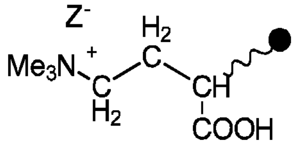 ;

wherein a wavy bond indicates that the radical's carbon atom attached through it may be in either of the R and the S configuration;
and wherein ● and Z are as defined in claim 2.

**4.** System according to claim 2, wherein the radical R is selected from

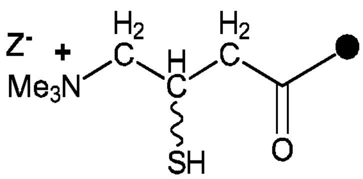 ; 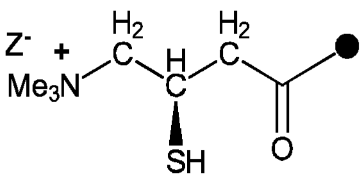 ;

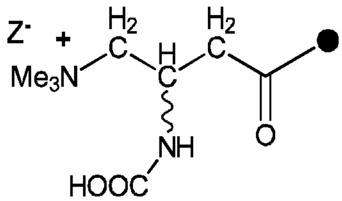 ; 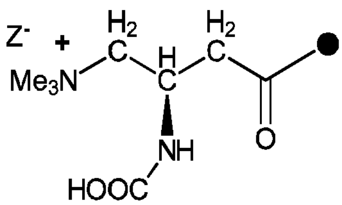 ;

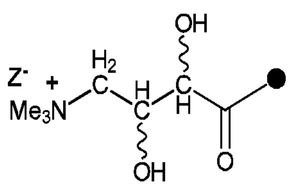

wherein a wavy bond indicates that the radical's carbon atom attached through it may be in either of the R and the S configuration;
and wherein ● and Z are as defined in claim 2.

**5.** System according to claims 1-2, wherein Ra, Rb and Rc each are methyl.

6. System according to claim 1-5, wherein the nanoparticles have an average size of about 100 nm.

7. System according to claims 1-6, wherein the polymer is selected form the list of a polyhydroxyalkanoate and a polyester.

8. System according to claim 7, wherein the polymer is a polyhydroxyalkanoate selected from poly(3-hydroxyproprionate), poly(3-hydroxybutyrate), poly(3-hydroxyvalerate) and copolymers of the same.

9. System according to claim 7, wherein the polymer is a polyester selected from polycaprolactone, polylactic acid, polyglycolic acid and copolymers of the same.

10. System according to claims 1-9, for use in transporting the bioactive molecule inside cells that express an organic cationic transporter.

11. System according to claim 10, wherein the bioactive molecule is selected from indomethacin, curcumin, acyclovir, coumarin-6, and (R)-3-(3-tetradecylureido)-4-(trimethylammonium)butanoate.

12. System according to claims 10 or 11, wherein the organic cationic transporter is selected from SLC22A4, SLC6A14 and SLC6A16.

**Patentansprüche**

1. Abgabesystem für bioaktive Moleküle, umfassend:

a) ein bioaktives Molekül; und
b) ein biokompatibles und/oder bioabbaubares Polymer in der Form von Nanopartikeln, die kovalent mit einem Rest der Formel R derivatisiert sind

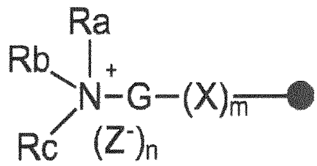

**R** ,

gegebenenfalls über einen Linker, und wobei:

● der Anknüpfungspunkt zu dem Polymer ist, oder, sofern ein Linker anwesend ist, zu dem Linker;
Ra, Rb und Rc gleich oder unterschiedlich sein können und ausgewählt sind aus Methyl und Ethyl;
G ein $C_1$-$C_5$ lineares, verzweigtes oder cyclisches Alkyl ist, gegebenenfalls substituiert mit einer oder mehreren Gruppe(n), ausgewählt aus Hydroxy, Carboxyl, Carboxylat, 1-Pyrrolidinyl, 1-Azetidinyl, 1-Aziridinyl, Sulfhydryl, -NRdRe und -N=NRf;
Rd, Re und Rf unabhängig ausgewählt sind aus Wasserstoff, Methyl, Ethyl und Propyl;
m null oder 1 sein kann;
-X- ausgewählt ist aus der Liste von -C(O)-, -NRg-, -S- und $-SO_2-$,
Rg ausgewählt ist aus Wasserstoff, Methyl, Ethyl und Propyl;
Z ein pharmazeutisch annehmbares Anion ist;
n null oder 1 sein kann, und nur null sein kann, sofern G ein Carboxylat trägt;
mit der Maßgabe, dass R nicht

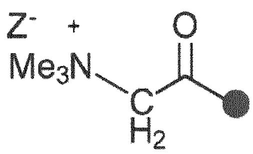

sein kann.

**2.** System gemäß Anspruch 1, wobei der Rest R ausgewählt ist aus Resten der Formel A, B und C

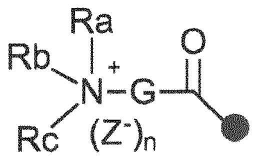

A

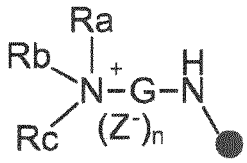

B

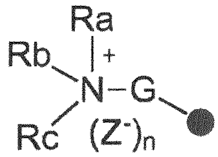

C ,

wobei ●, Ra, Rib, Rc, G, Z und n wie in Anspruch 1 definiert sind.

**3.** System gemäß Anspruch 2, wobei der Rest R ausgewählt ist aus

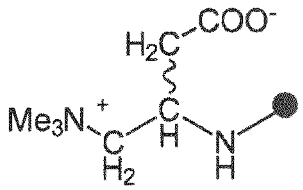 ; 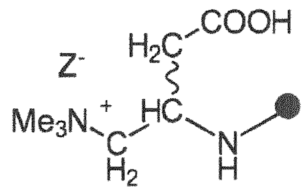 ;

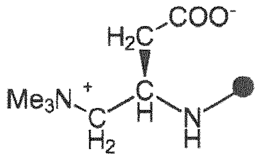 ; 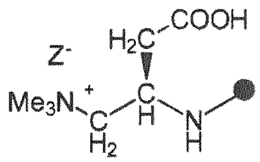 ; 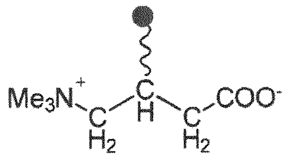 ;

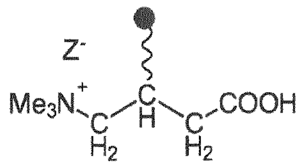 ; 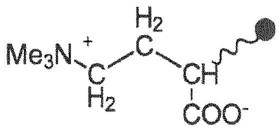 ; 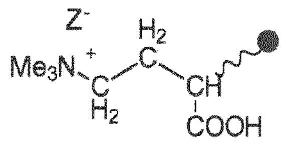 ;

wobei eine geschlängelte Bindung anzeigt, dass das Kohlenstoffatom des darüber verknüpften Restes entweder in der R- oder in der S-Konfiguration vorliegen kann;
und wobei ● und Z wie in Anspruch 2 definiert sind.

**4.** System gemäß Anspruch 2, wobei der Rest R ausgewählt ist aus

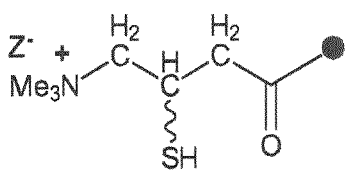 ; 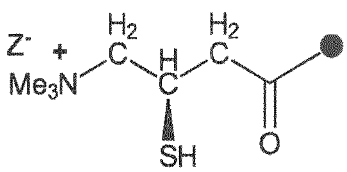 ;

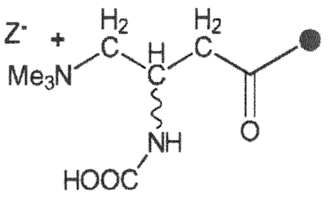 ; 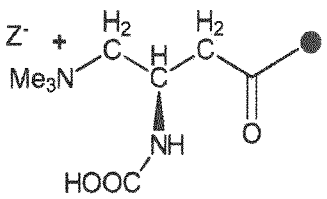 ;

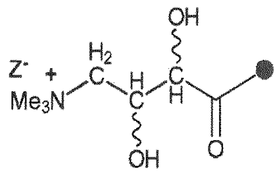

wobei eine geschlängelte Bindung anzeigt, dass das Kohlenstoffatom des darüber verknüpften Restes entweder in der R- oder in der S-Konfiguration vorliegen kann;
und wobei ● und Z wie in Anspruch 2 definiert sind.

**5.** System gemäß Ansprüchen 1-2, wobei Ra, Rb und Rc jeweils Methyl sind.

**6.** System gemäß Ansprüchen 1-5, wobei die Nanopartikel eine durchschnittliche Größe von etwa 100 nm haben.

**7.** System gemäß Ansprüchen 1-6, wobei das Polymer ausgewählt ist aus der Liste aus einem Polyhydroxyalkanoat und einem Polyester.

**8.** System gemäß Anspruch 7, wobei das Polymer ein Polyhydroxyalkanoat ist, ausgewählt aus Poly(3-hydroxyproprionat), Poly(3-hydroxybutyrat), Poly(3-hydroxyvalerat) sowie Copolymeren der vorgenannten.

**9.** System gemäß Anspruch 7, wobei das Polymer ein Polyester ist, ausgewählt aus Polycaprolacton, Polymilchsäure, Polyglycolsäure und Copolymeren der vorgenannten.

**10.** System gemäß Ansprüchen 1-9 zur Verwendung im Transport des bioaktiven Moleküls innerhalb von Zellen, welche einen organischen kationischen Transporter exprimieren.

**11.** System gemäß Anspruch 10, wobei das bioaktive Molekül ausgewählt ist aus Indomethacin, Curcumin, Acyclovir, Coumarin-6 und (R)-3-(3-Tetradecylureido)-4-(trimethylammonium)butanoat.

**12.** System gemäß Anspruch 10 oder 11, wobei der organische kationische Transporter ausgewählt ist aus SLC22A4, SLC6A14 und SLC6A16.

## Revendications

**1.** Système d'administration de molécule bioactive qui comprend :

a) une molécule bioactive ; et
b) un polymère biocompatible et/ou biodégradable sous la forme de nanoparticules dérivées de manière covalente d'un radical de formule R

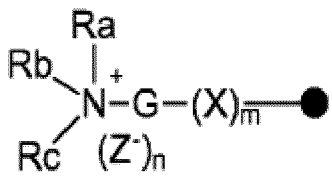

**R**

de manière facultative par l'intermédiaire d'un lieur ; et dans lequel :

• représente le point de fixation au polymère, ou, lorsqu'un lieur est présent, au lieur ;
Ra, Rb et Rc peuvent être identiques ou différents et sont choisis parmi un groupe méthyle et un groupe éthyle ;
G représente un groupe alkyle en $C_1$-$C_5$ à chaîne droite, à chaîne ramifiée ou cyclique, de manière facultative substitué avec un ou plusieurs groupes choisis parmi un groupe hydroxyle, carboxyle, carboxylate, 1-pyrrolidinyle, 1-azétidinyle, 1-aziridinyle, sulfhydryle, -NRdRe et -N=NRf ;
Rd, Re et Rf sont choisis de manière indépendante parmi un atome d'hydrogène, un groupe méthyle, éthyle et propyle ;
m peut être égal à zéro ou à 1;
-X- est choisi parmi la liste qui comprend -C(O)-, -NRg-, -S- et $-SO_2-$;
Rg est choisi parmi un atome d'hydrogène, un groupe méthyle, éthyle et propyle ;
Z représente un anion pharmaceutiquement acceptable ;
n peut être égal à zéro ou à 1, et il ne peut être égal qu'à zéro lorsque G porte un groupe carboxylate ;
avec cette réserve que R ne peut pas représenter :

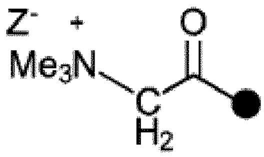

**2.** Système selon la revendication 1, dans lequel le radical R est choisi parmi des radicaux de formule A, B et C :

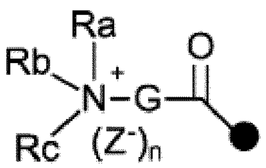

A

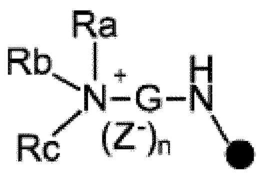

B

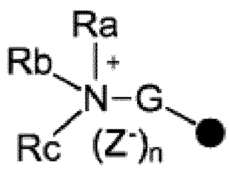

C

dans lesquelles ●, Ra, Rib, Rc, G, Z et n sont tels que définis dans la revendication 1.

**3.** Système selon la revendication 2, dans lequel le radical R est choisi parmi :

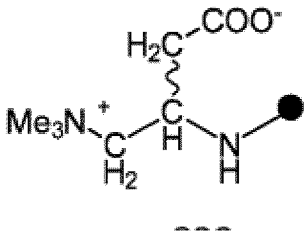 ; 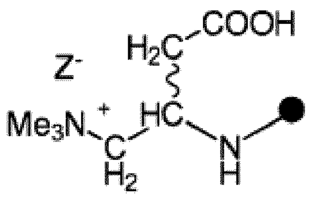 ;

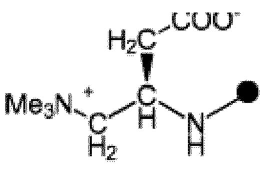 ; 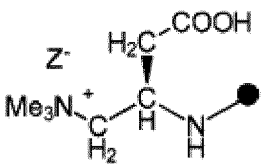 ; 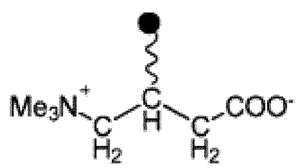 ;

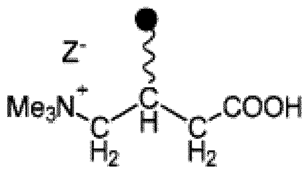 ; 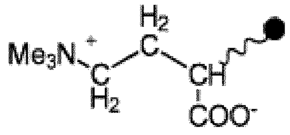 ; 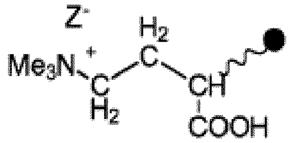 ;

dans lesquelles une liaison ondulée désigne le fait que l'atome de carbone du radical fixé par l'intermédiaire de cette dernière peut être soit dans la configuration R, soit dans la configuration S ;
et dans lesquelles • et Z sont tels que définis dans la revendication 2.

4. Système selon la revendication 2, dans lequel le radical R est choisi parmi :

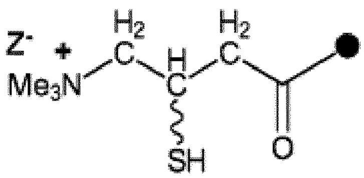 ; 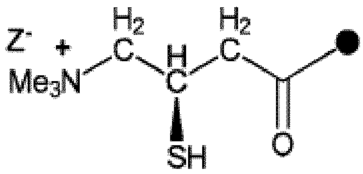 ;

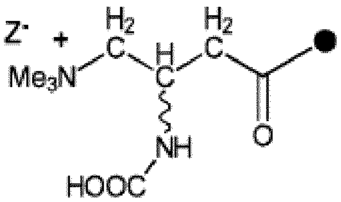 ; 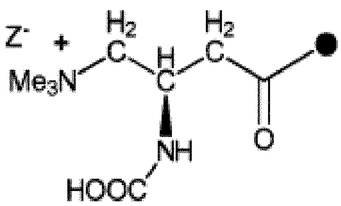 ;

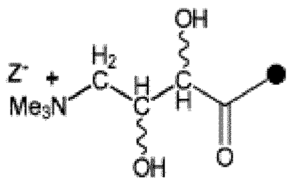

dans lesquelles une liaison ondulée désigne le fait que l'atome de carbone du radical fixé par l'intermédiaire de cette dernière peut être soit dans la configuration R, soit dans la configuration S ;
et dans lesquelles • et Z sont tels que définis dans la revendication 2.

5. Système selon les revendications 1 à 2, dans lequel Ra, Rb et Rc représentent chacun un groupe méthyle.

6. Système selon les revendications 1 à 5, dans lequel les nanoparticules possèdent une dimension moyenne d'environ 100 nm.

7. Système selon les revendications 1 à 6, dans lequel le polymère est choisi parmi la liste qui comprend un polyhydroxyalcanoate et un polyester.

8. Système selon la revendication 7, dans lequel le polymère est un polyhydroxyalcanoate choisi parmi un poly(3-hydroxyproprionate), un poly(3-hydroxybutyrate), un poly(3-hydroxyvalérate) et des copolymères de ces derniers.

9. Système selon la revendication 7, dans lequel le polymère est un polyester choisi parmi la polycaprolactone, l'acide polylactique, l'acide polyglycolique et des copolymères de ces derniers.

10. Système selon les revendications 1 à 9, pour son utilisation dans le transport de la molécule bioactive à l'intérieur de cellules qui expriment un transporteur cationique organique.

11. Système selon la revendication 10, dans lequel la molécule bioactive est choisie parmi l'indométhacine, la curcumine,

l'acyclovir, la coumarine-6, et le (R) -3-(3-tétradécyluréido)-4-(triméthylammonium)butanoate.

**12.** Système selon la revendication 10 ou 11, dans lequel le transporteur cationique organique est choisi parmi SLC22A4, SLC6A14 et SLC6A16.

Figure 1

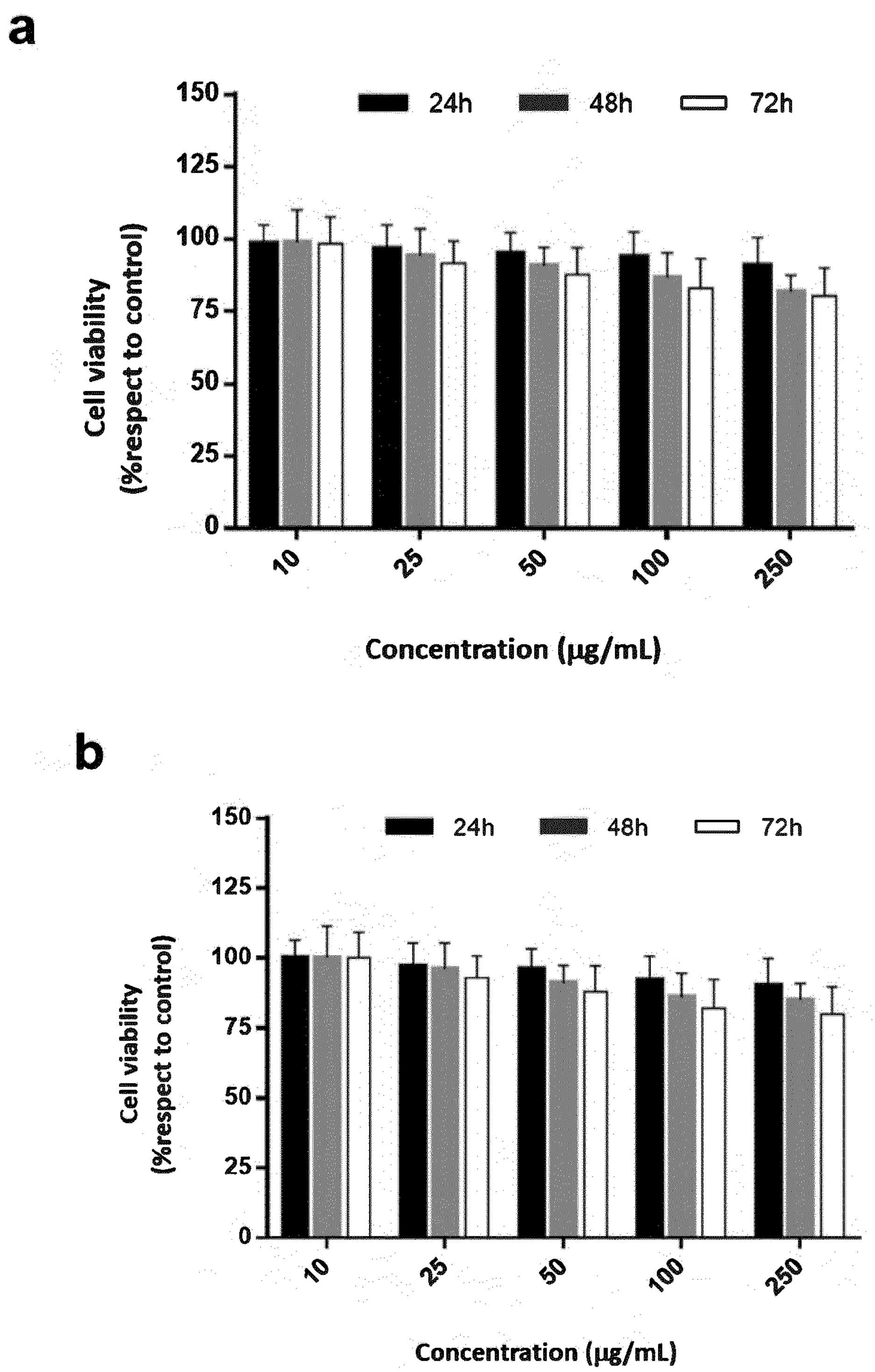
a
150
125
100
75
50
25
0
24h
48h
72h
Cell viability
(%respect to control)
10
25
50
100
250
Concentration (µg/mL)
b
150
125
100
75
50
25
0
24h
48h
72h
Cell viability
(%respect to control)
10
25
50
100
250
Concentration (µg/mL)

Figure 1 (continued)

C

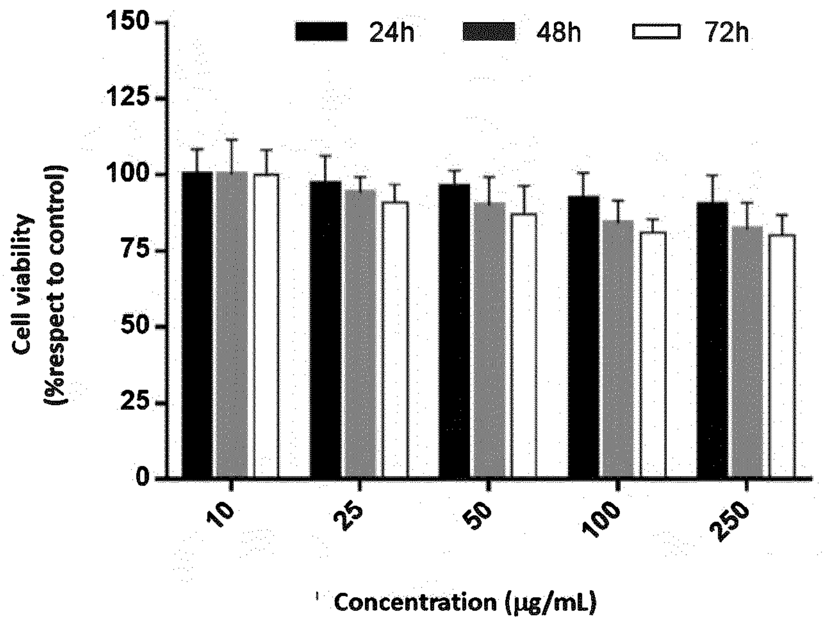

Figure 2

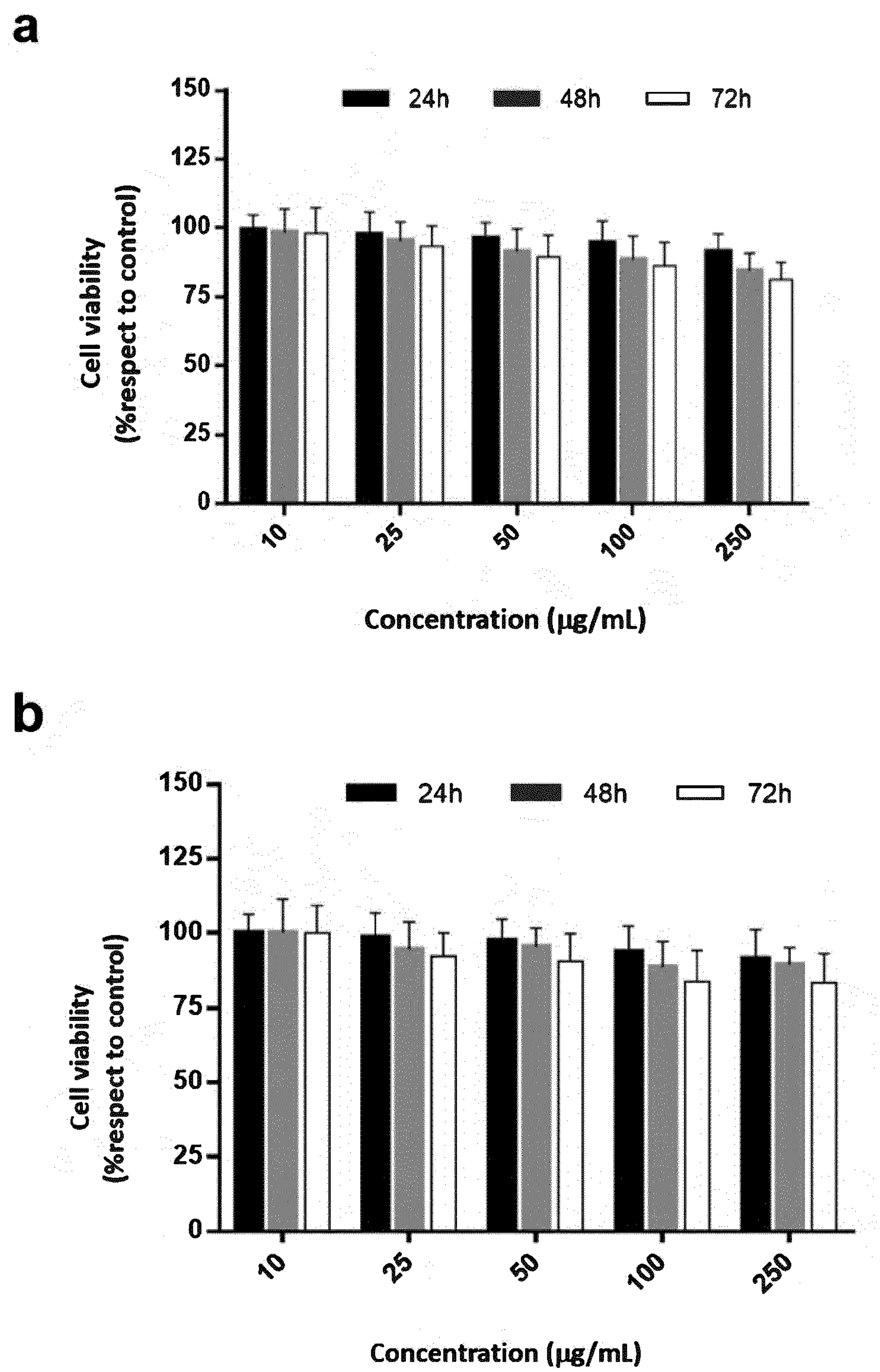
a
150
125
100
75
50
25
0
24h
48h
72h
Cell viability
(%respect to control)
10
25
50
100
250
Concentration (µg/mL)
b
150
125
100
75
50
25
0
24h
48h
72h
Cell viability
(%respect to control)
10
25
50
100
250
Concentration (µg/mL)

Figure 2 (continued)

C

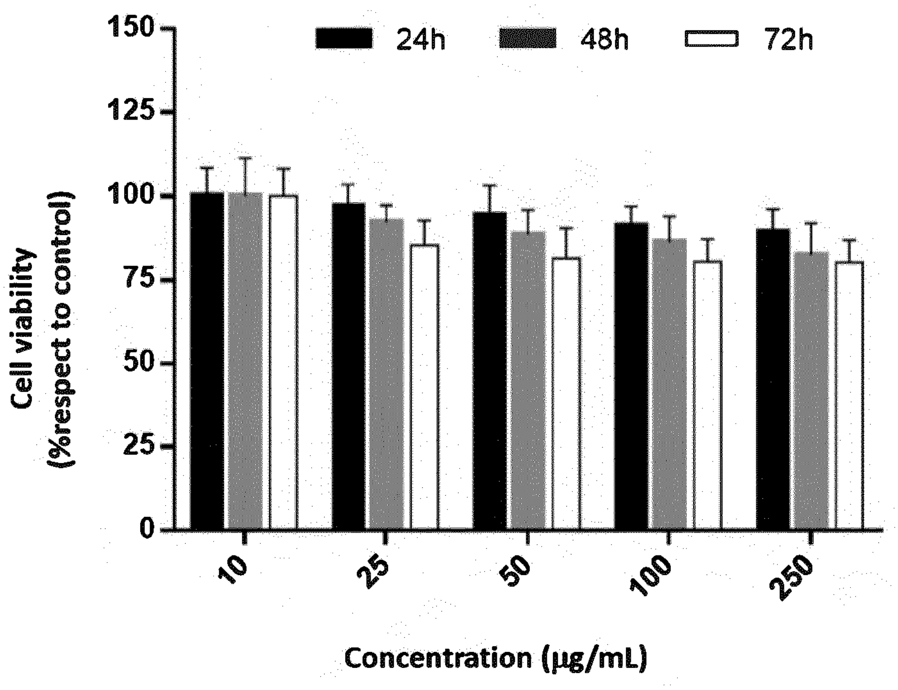

Figure 3

a

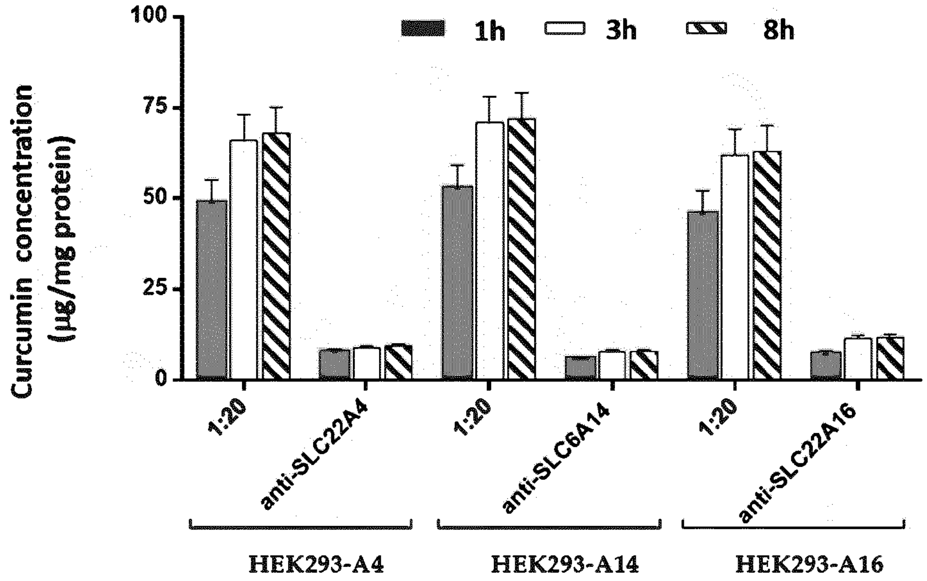
1h
3h
8h
Curcumin concentration
(µg/mg protein)
100
75
50
25
0
1:20
anti-SLC22A4
1:20
anti-SLC6A14
1:20
anti-SLC22A16
HEK293-A4
HEK293-A14
HEK293-A16

b

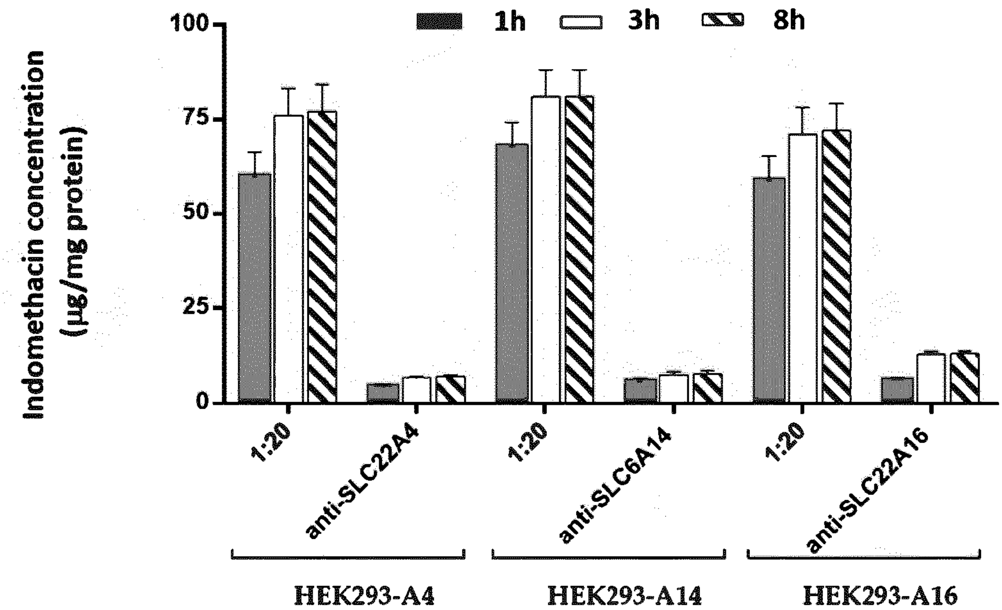
1h
3h
8h
Indomethacin concentration
(µg/mg protein)
100
75
50
25
0
1:20
anti-SLC22A4
1:20
anti-SLC6A14
1:20
anti-SLC22A16
HEK293-A4
HEK293-A14
HEK293-A16

Figure 3 (continued)

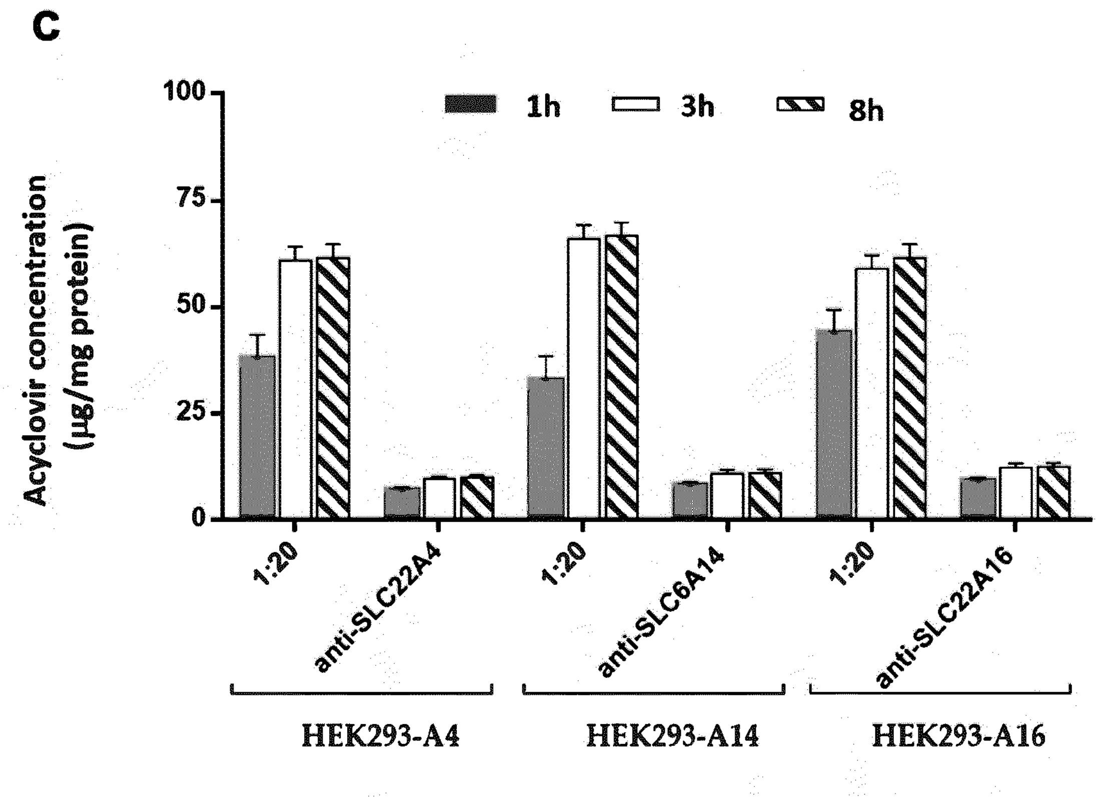

Figure 4

a

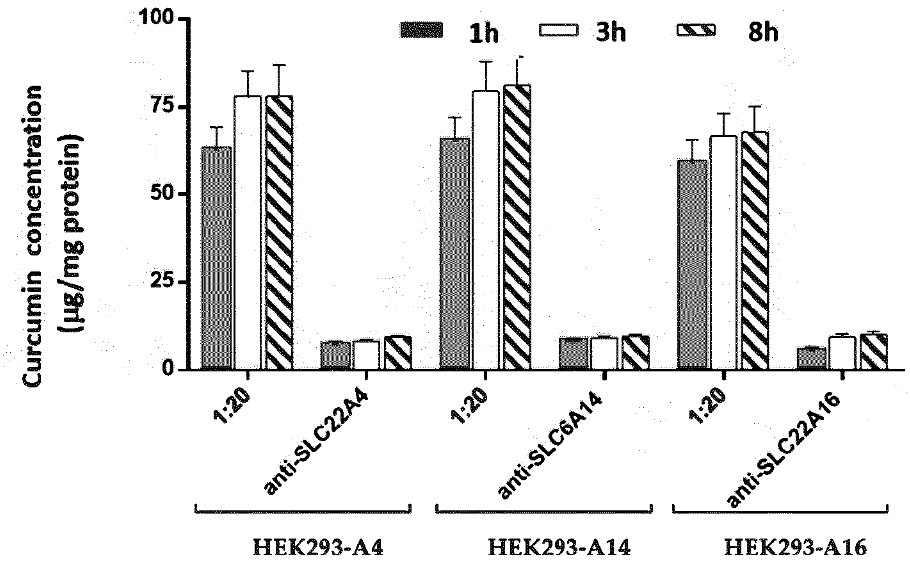
1h
3h
8h
Curcumin concentration (µg/mg protein)
100
75
50
25
0
1:20
anti-SLC22A4
1:20
anti-SLC6A14
1:20
anti-SLC22A16
HEK293-A4
HEK293-A14
HEK293-A16

b

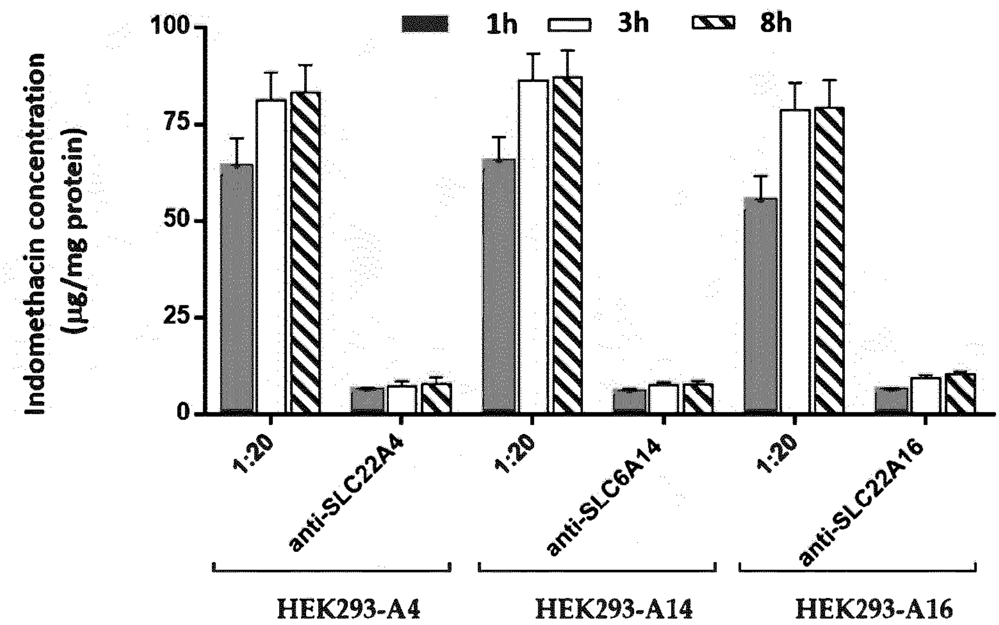
1h
3h
8h
Indomethacin concentration (µg/mg protein)
100
75
50
25
0
1:20
anti-SLC22A4
1:20
anti-SLC6A14
1:20
anti-SLC22A16
HEK293-A4
HEK293-A14
HEK293-A16

Figure 4 (continued)

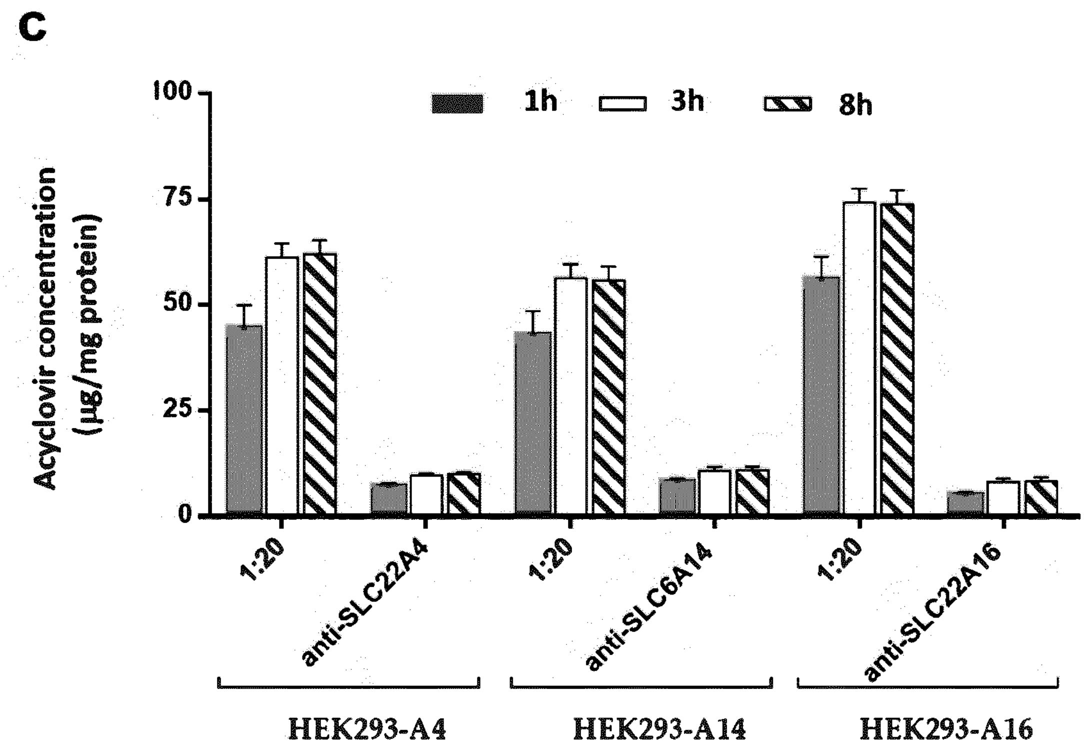

Figure 5

a)

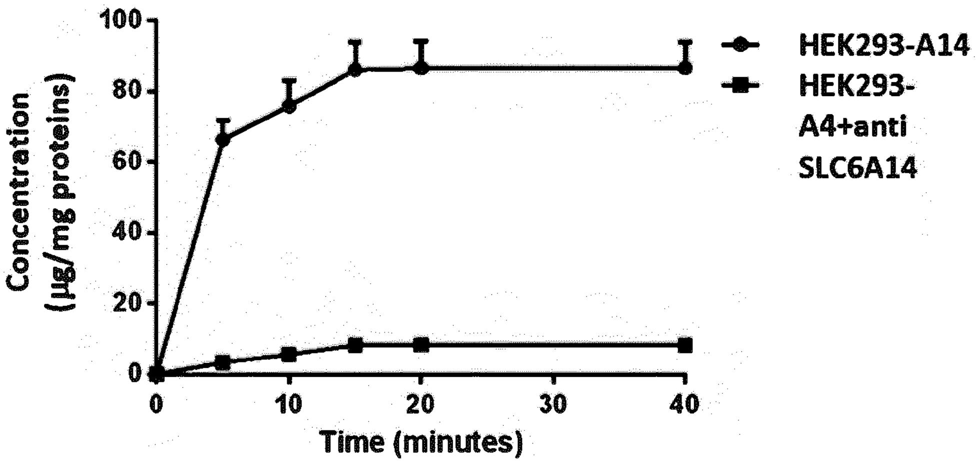
100
80
60
40
20
0
Concentration (µg/mg proteins)
0
10
20
30
40
Time (minutes)
HEK293-A14
HEK293-A4+anti SLC6A14

b)

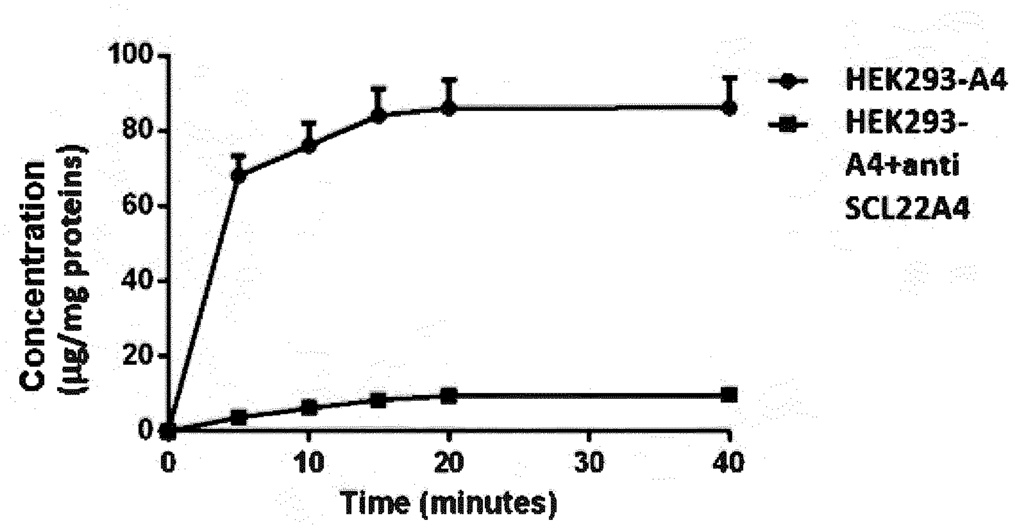
100
80
60
40
20
0
Concentration (µg/mg proteins)
0
10
20
30
40
Time (minutes)
HEK293-A4
HEK293-A4+anti SCL22A4

Figure 5 (continued)

c)

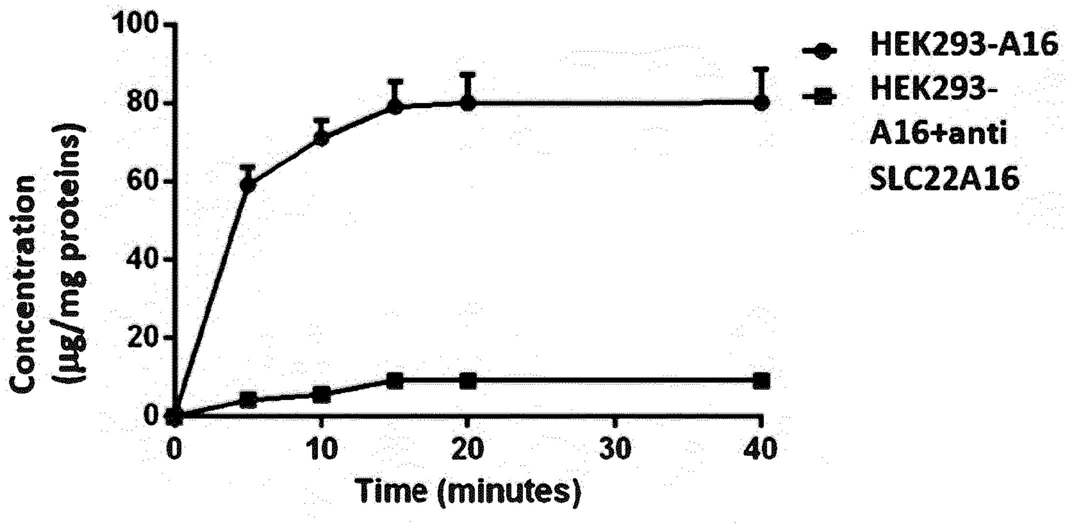

Figure 6

a)

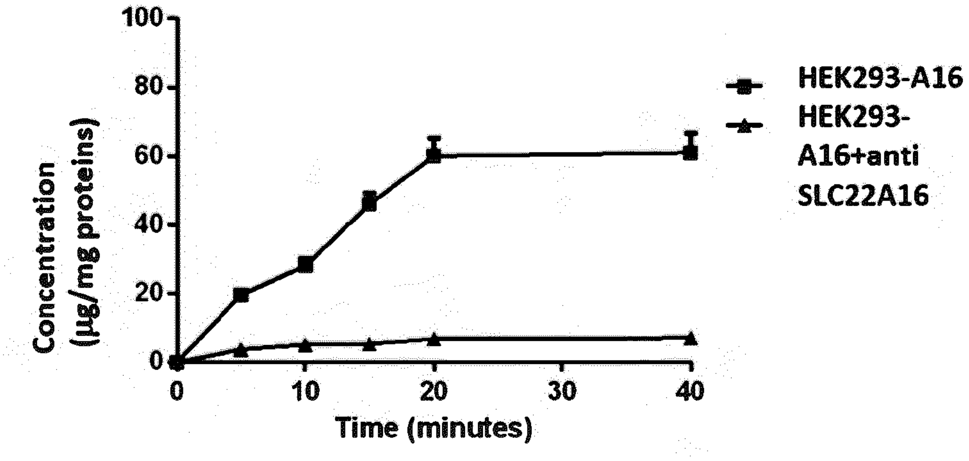
100
80
60
40
20
0
Concentration (µg/mg proteins)
0
10
20
30
40
Time (minutes)
HEK293-A16
HEK293-A16+anti SLC22A16

b)

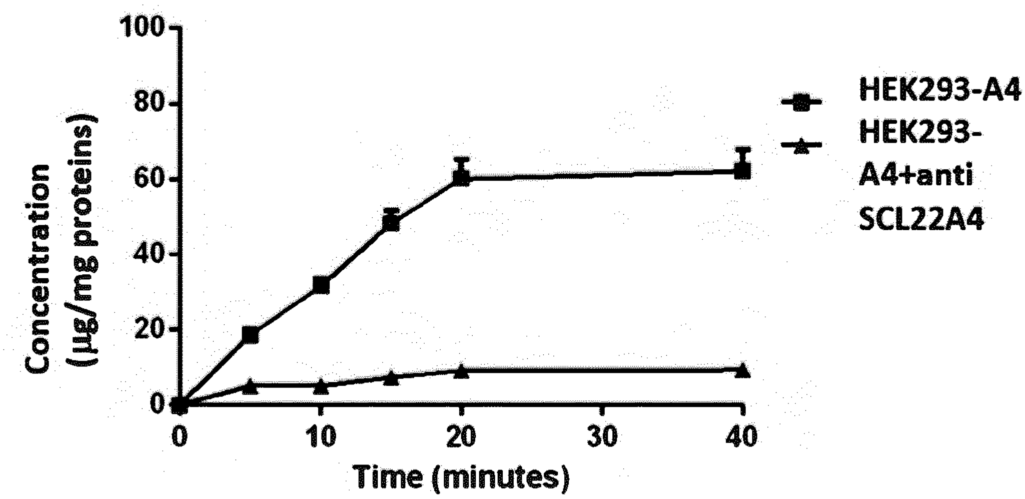
100
80
60
40
20
0
Concentration (µg/mg proteins)
0
10
20
30
40
Time (minutes)
HEK293-A4
HEK293-A4+anti SCL22A4

Figure 6 (continued)

c)

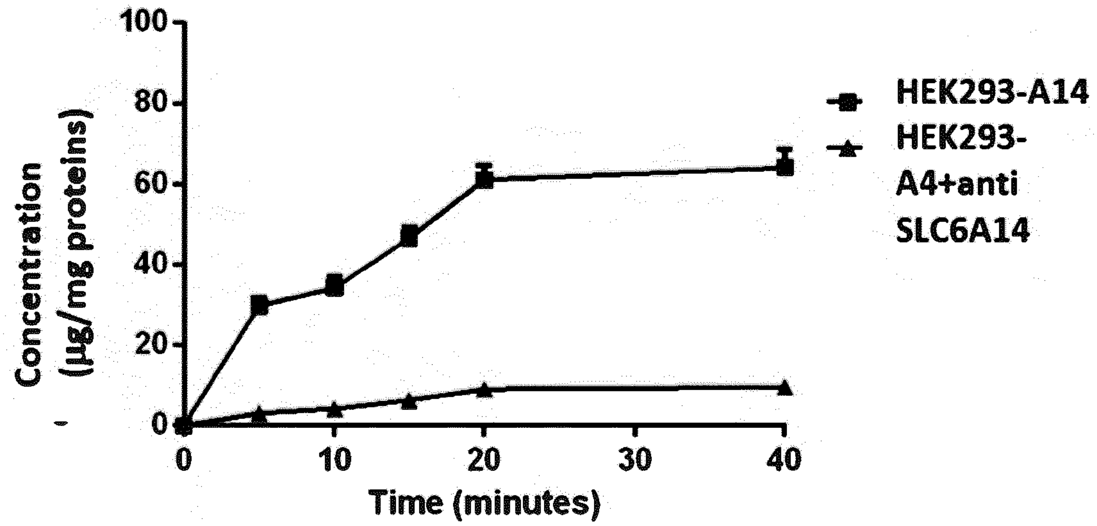

Figure 7

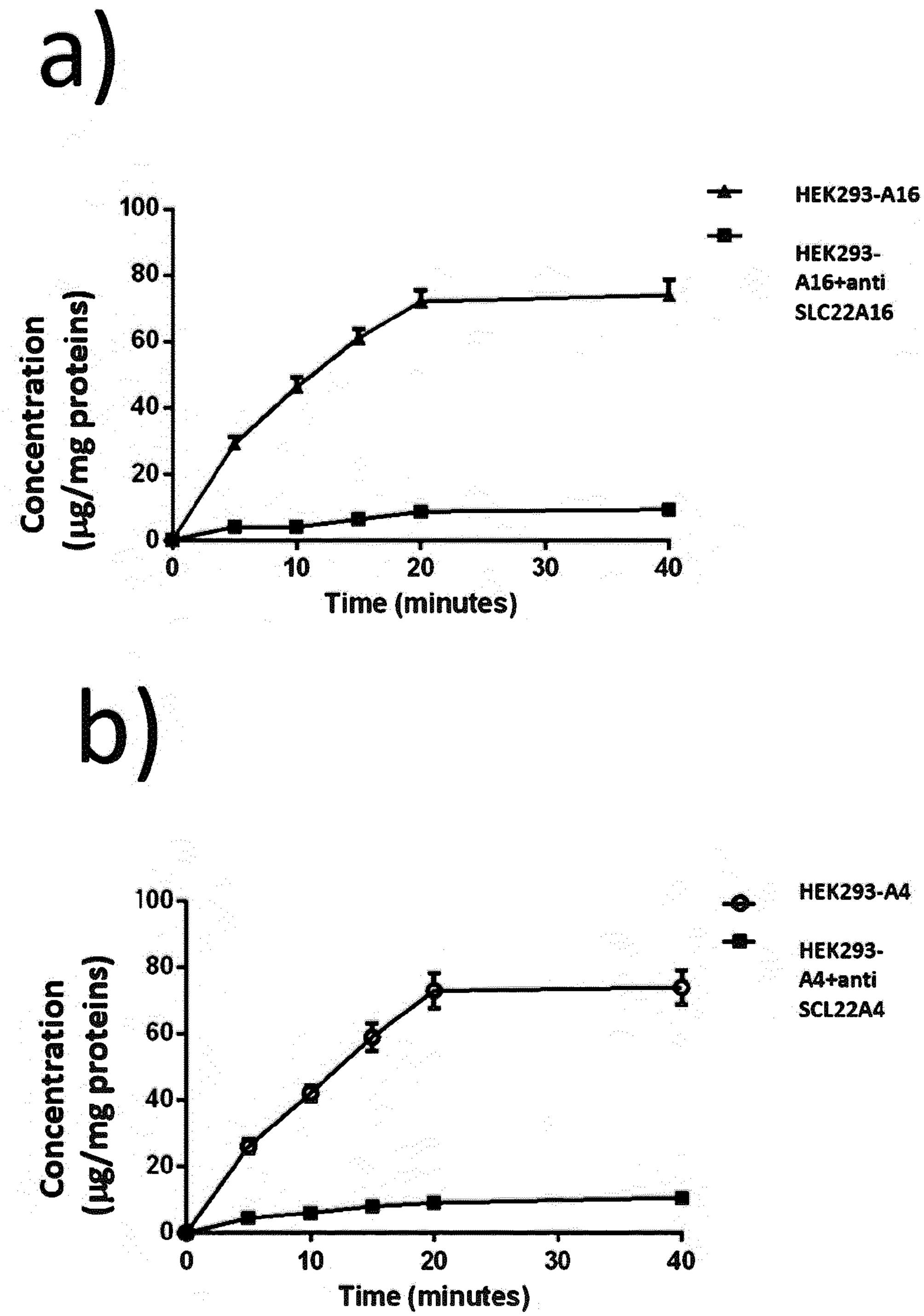
a)
HEK293-A16
HEK293-A16+anti SLC22A16
Concentration (µg/mg proteins)
100
80
60
40
20
0
0
10
20
30
40
Time (minutes)
b)
HEK293-A4
HEK293-A4+anti SCL22A4
Concentration (µg/mg proteins)
100
80
60
40
20
0
0
10
20
30
40
Time (minutes)

Figure 7 (continued)

c)

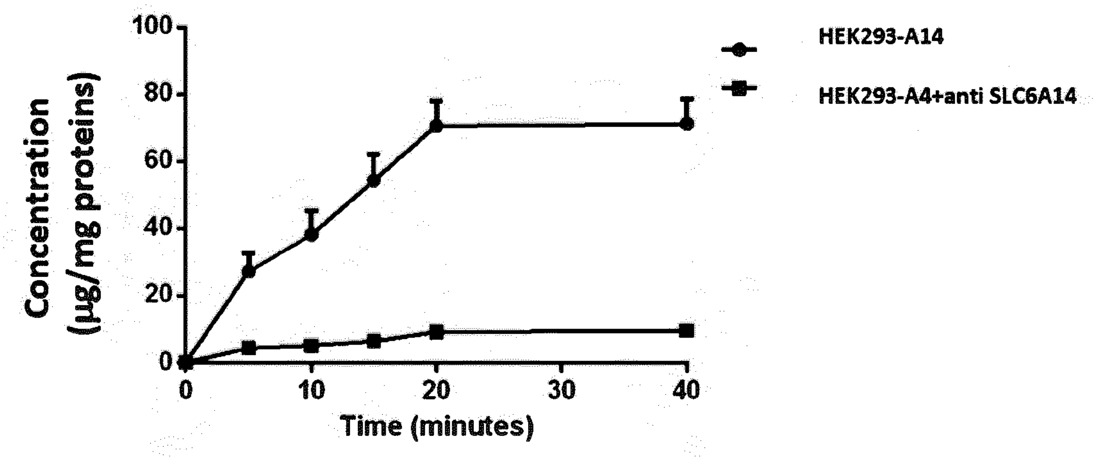

Figure 8

a)

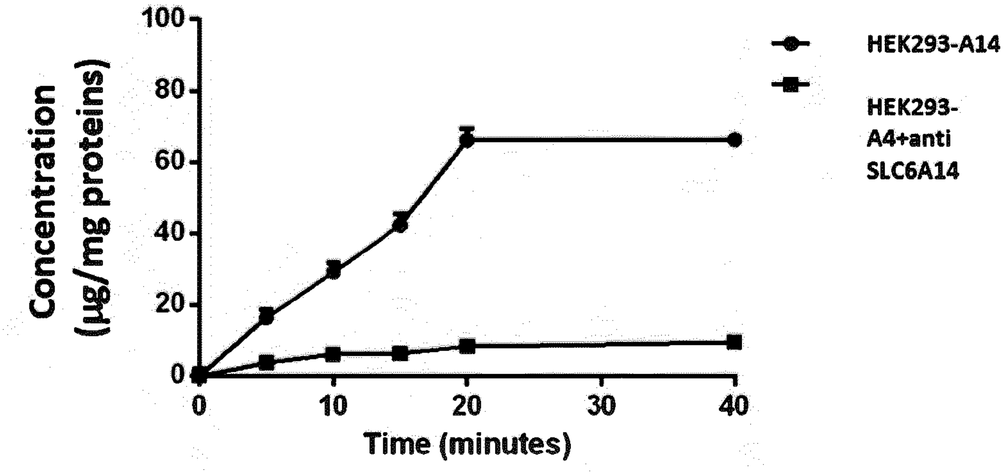
Concentration (µg/mg proteins)
100
80
60
40
20
0
0
10
20
30
40
Time (minutes)
HEK293-A14
HEK293-A4+anti SLC6A14

b)

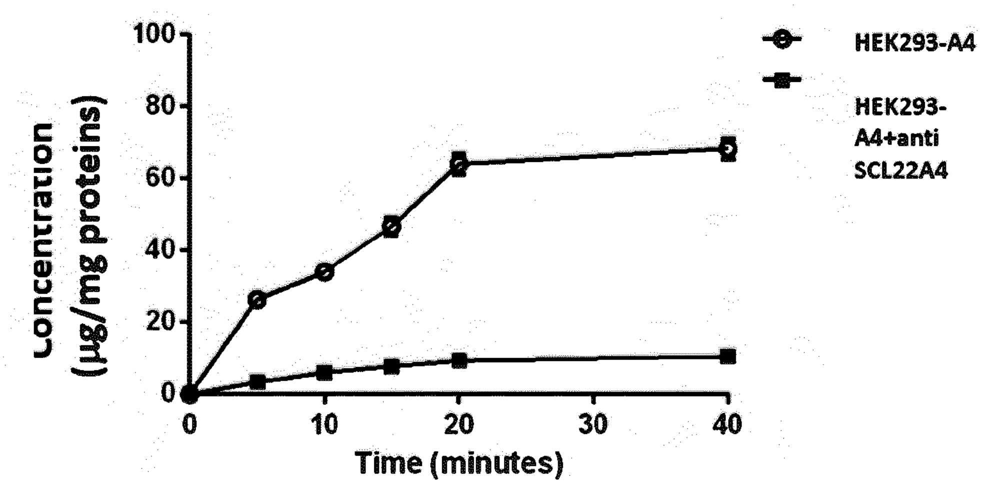
Concentration (µg/mg proteins)
100
80
60
40
20
0
0
10
20
30
40
Time (minutes)
HEK293-A4
HEK293-A4+anti SCL22A4

Figure 8 (continued)

c)

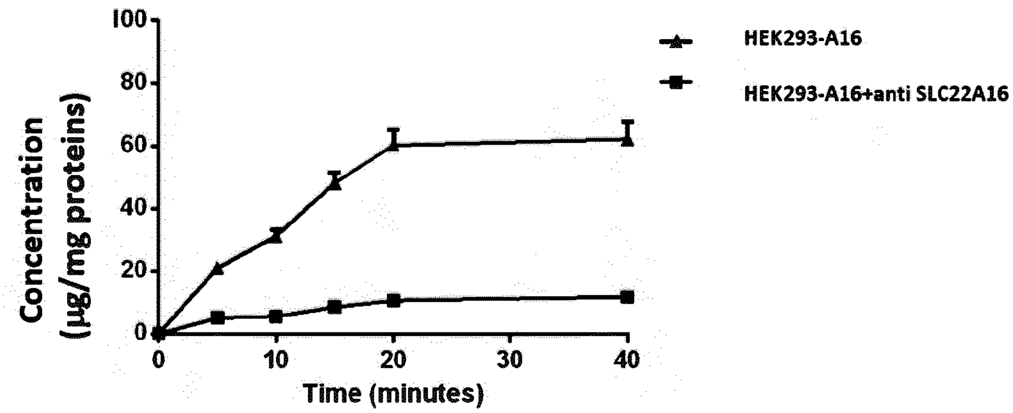

Figure 9

a)

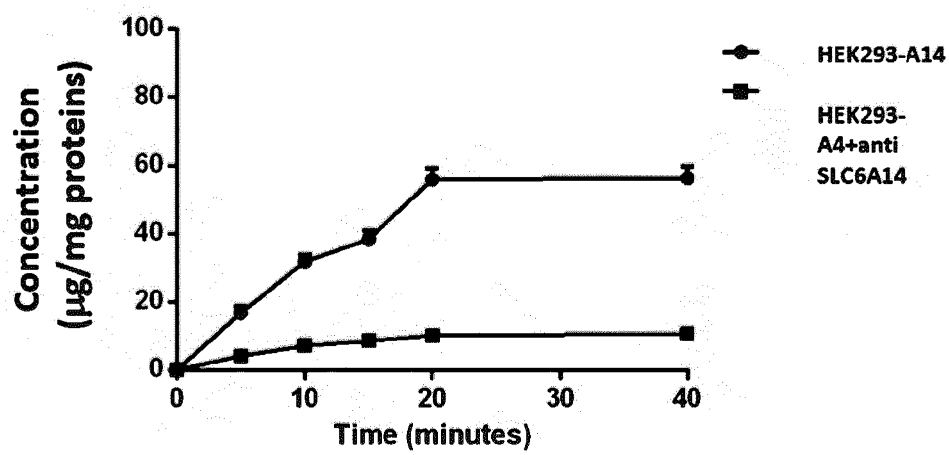
100
80
60
40
20
0
Concentration
(µg/mg proteins)
0
10
20
30
40
Time (minutes)
HEK293-A14
HEK293-
A4+anti
SLC6A14

b)

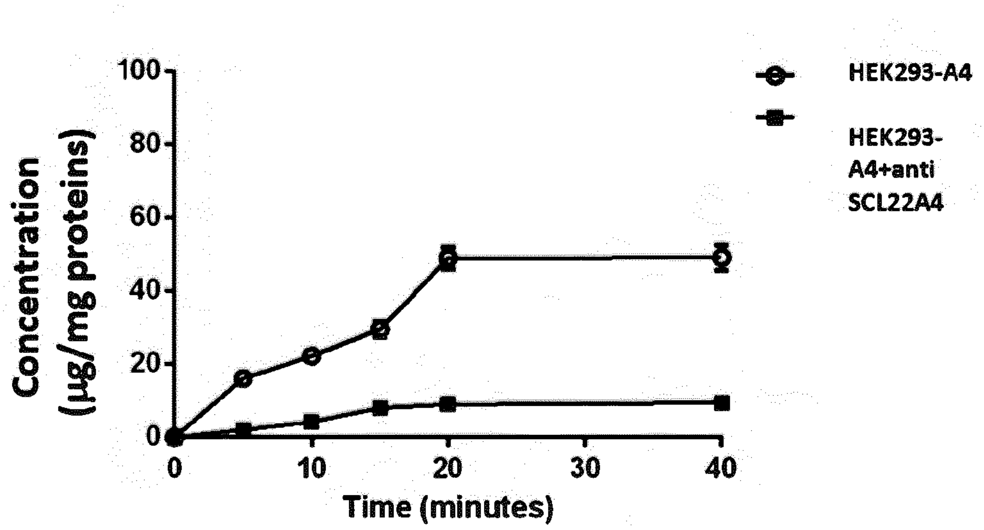
100
80
60
40
20
0
Concentration
(µg/mg proteins)
0
10
20
30
40
Time (minutes)
HEK293-A4
HEK293-
A4+anti
SCL22A4

Figure 9 (continued)

c)

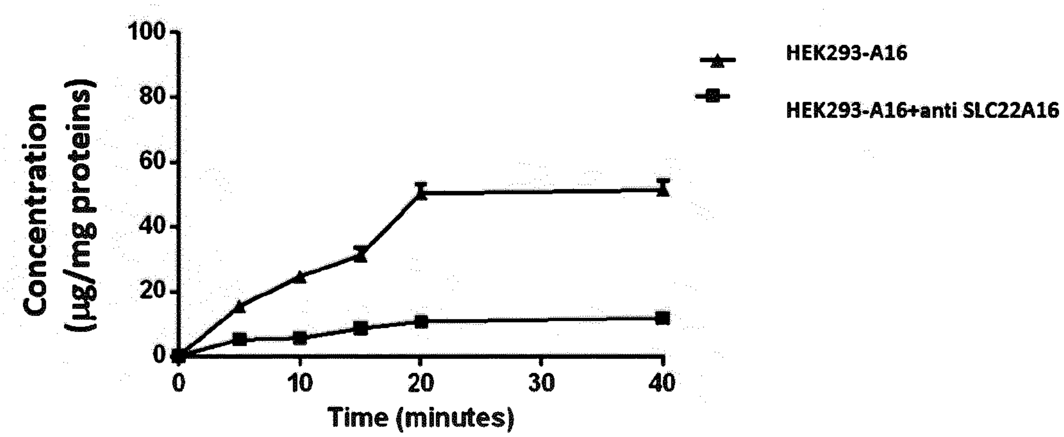

Figure 10

a

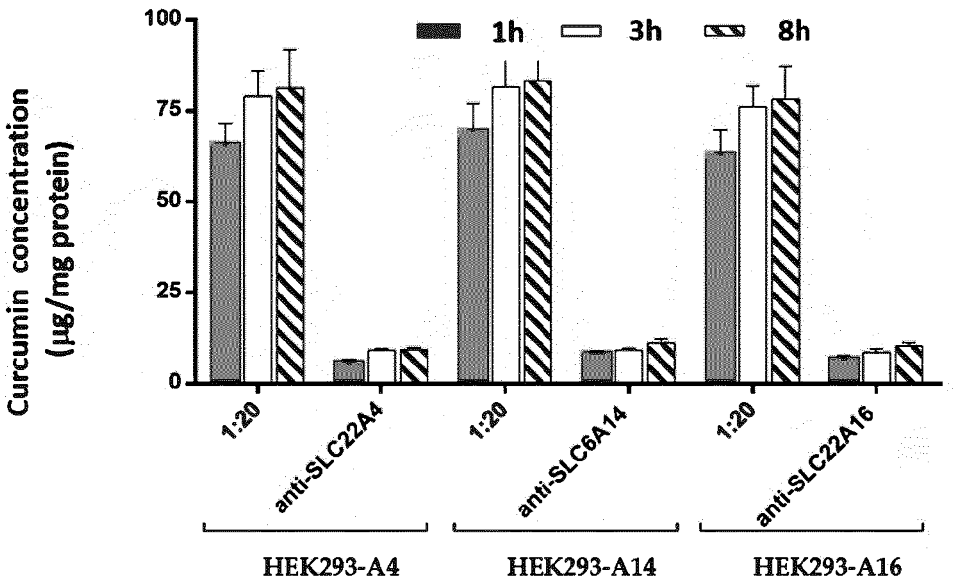
100
75
50
25
0
1h
3h
8h
Curcumin concentration
(µg/mg protein)
1:20
anti-SLC22A4
1:20
anti-SLC6A14
1:20
anti-SLC22A16
HEK293-A4
HEK293-A14
HEK293-A16

b

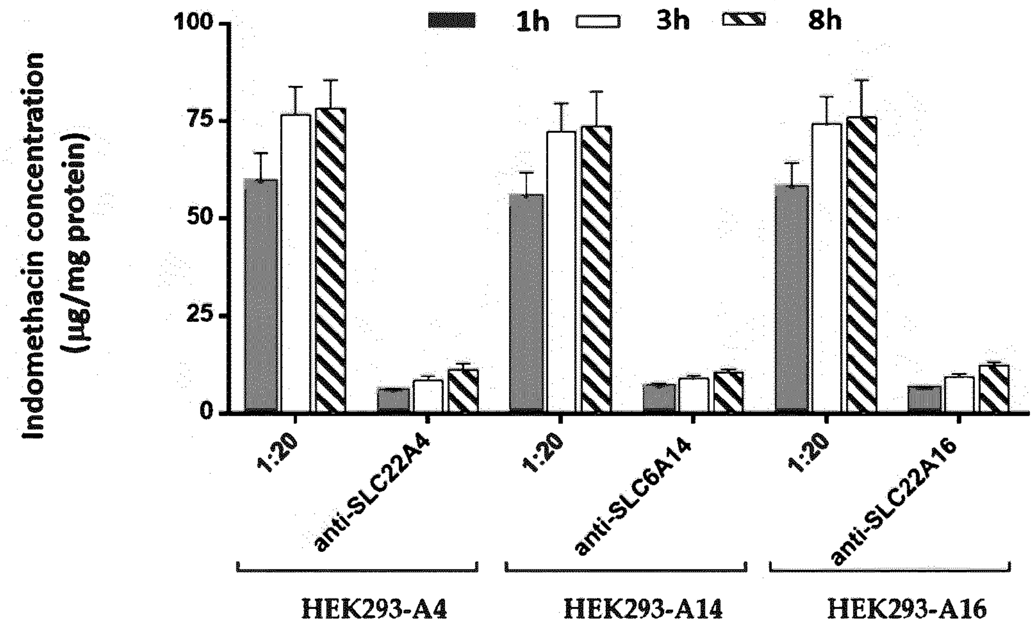
100
75
50
25
0
1h
3h
8h
Indomethacin concentration
(µg/mg protein)
1:20
anti-SLC22A4
1:20
anti-SLC6A14
1:20
anti-SLC22A16
HEK293-A4
HEK293-A14
HEK293-A16

Figure 10 (continued)

C

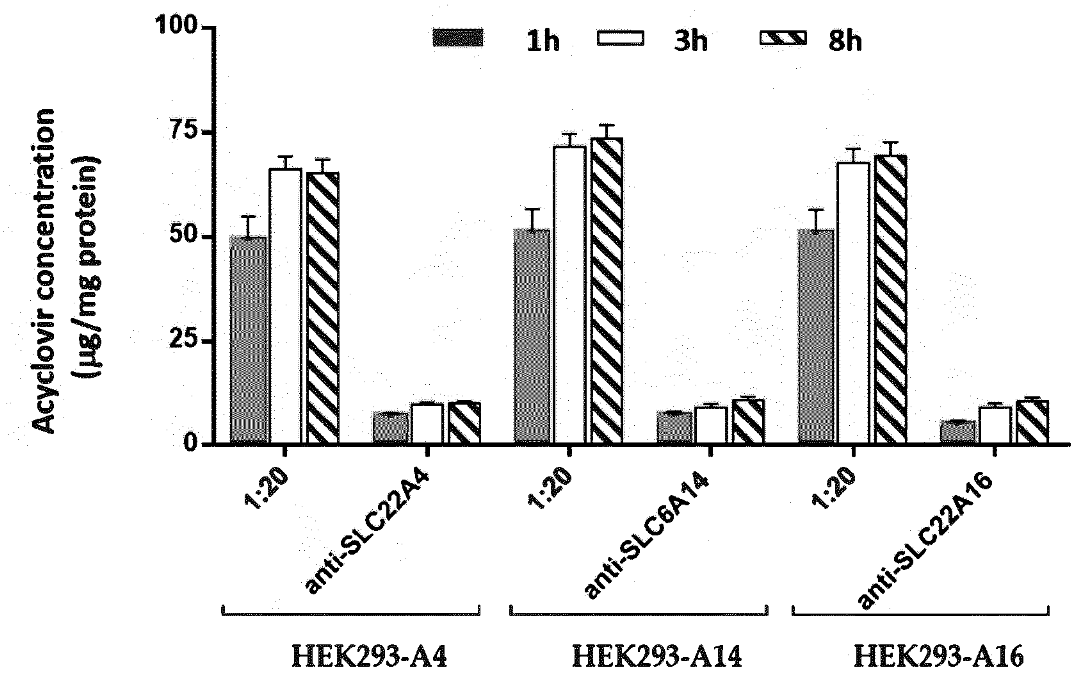

Figure 11

a)

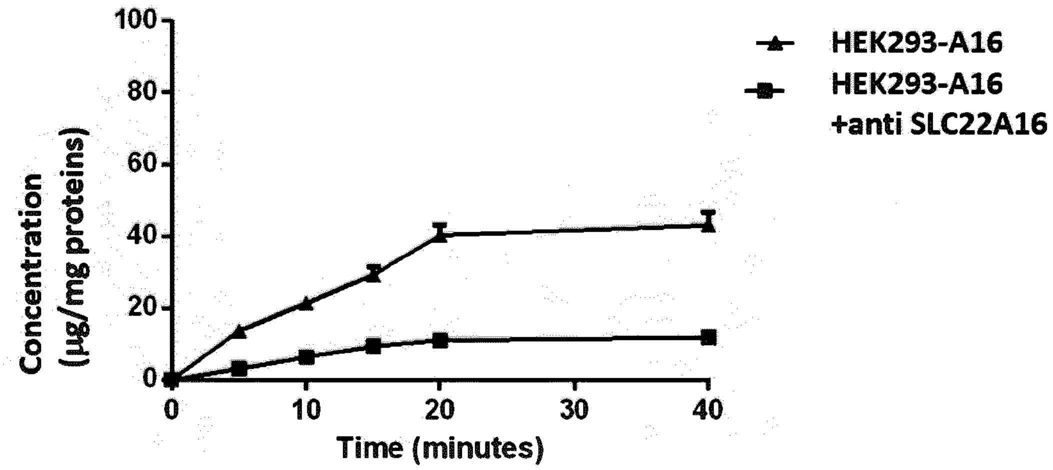
100
80
60
40
20
0
Concentration
(µg/mg proteins)
0
10
20
30
40
Time (minutes)
HEK293-A16
HEK293-A16
+anti SLC22A16

b)

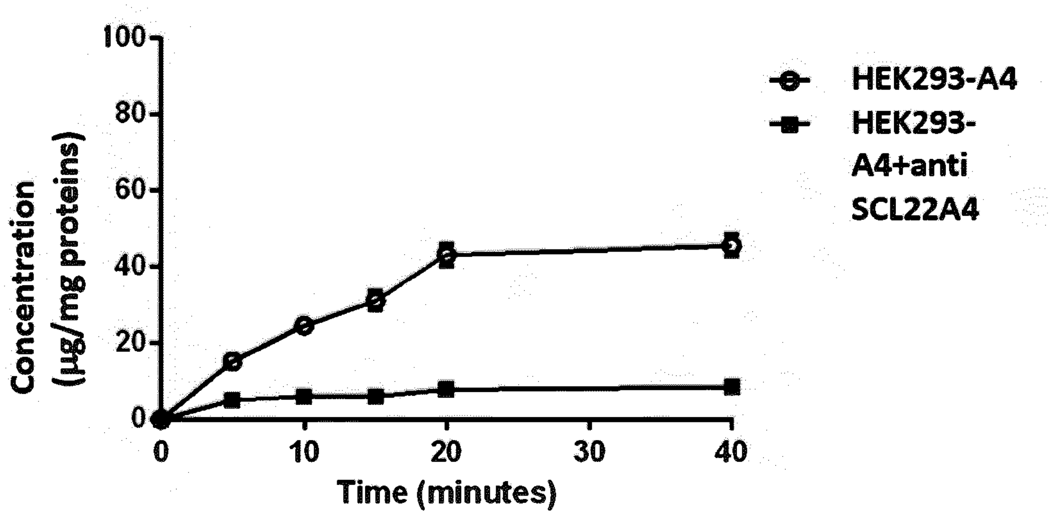
100
80
60
40
20
0
Concentration
(µg/mg proteins)
0
10
20
30
40
Time (minutes)
HEK293-A4
HEK293-
A4+anti
SCL22A4

Figure 11 (continued)

c)

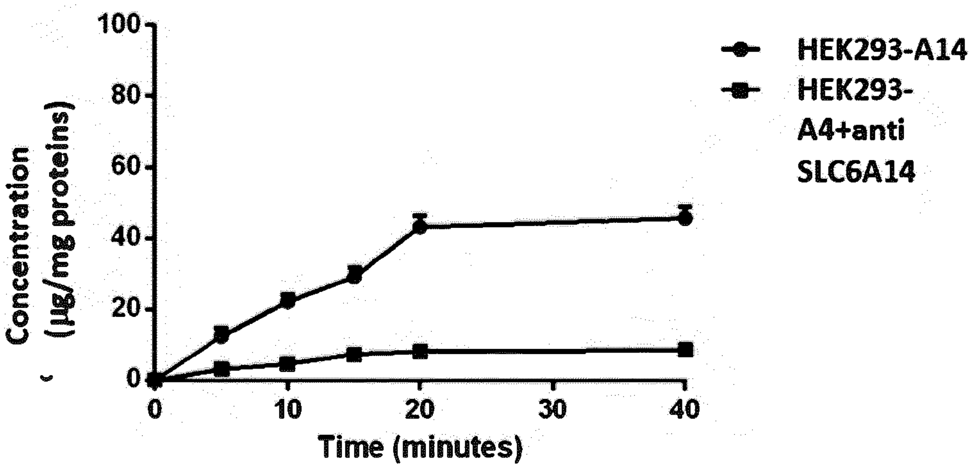

Figure 12

A

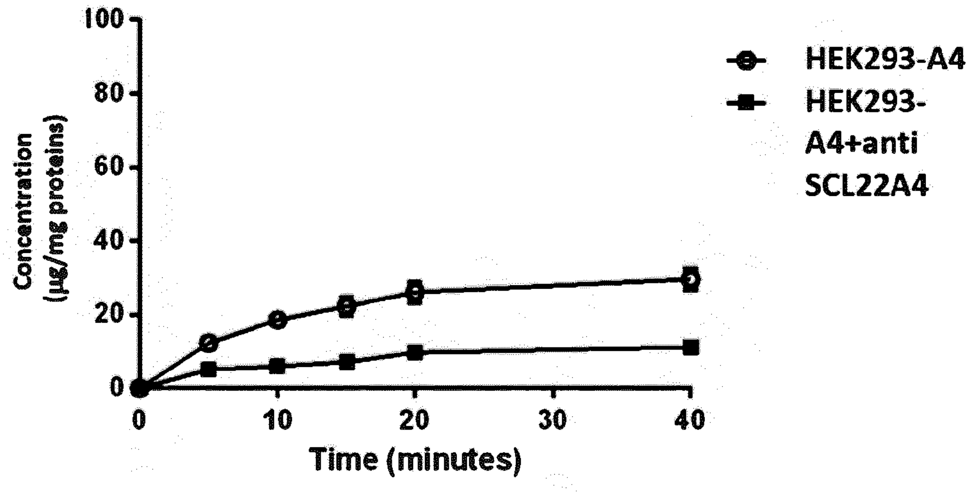
100
80
60
40
20
0
Concentration (µg/mg proteins)
0
10
20
30
40
Time (minutes)
HEK293-A4
HEK293-A4+anti SCL22A4

B

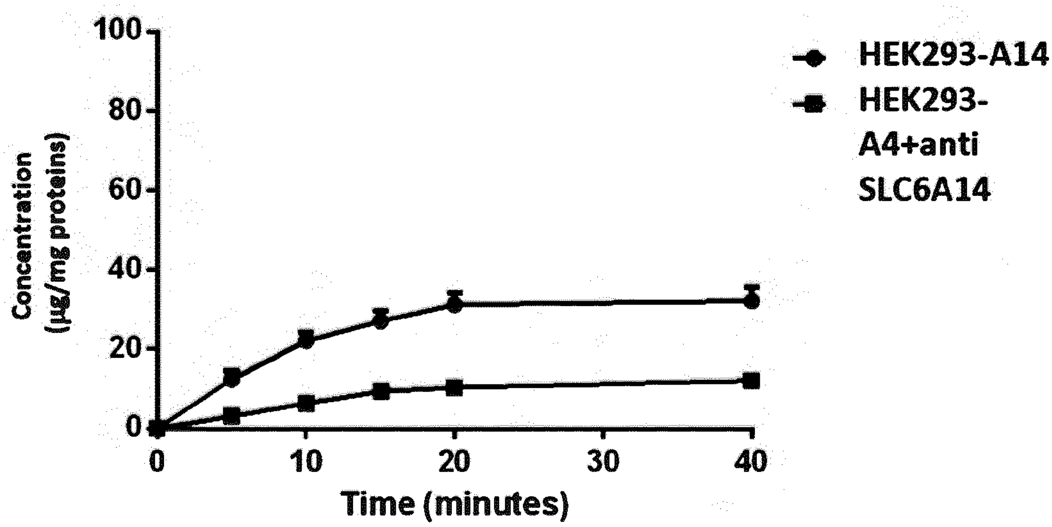
100
80
60
40
20
0
Concentration (µg/mg proteins)
0
10
20
30
40
Time (minutes)
HEK293-A14
HEK293-A4+anti SLC6A14

Figure 12 (continued)

C

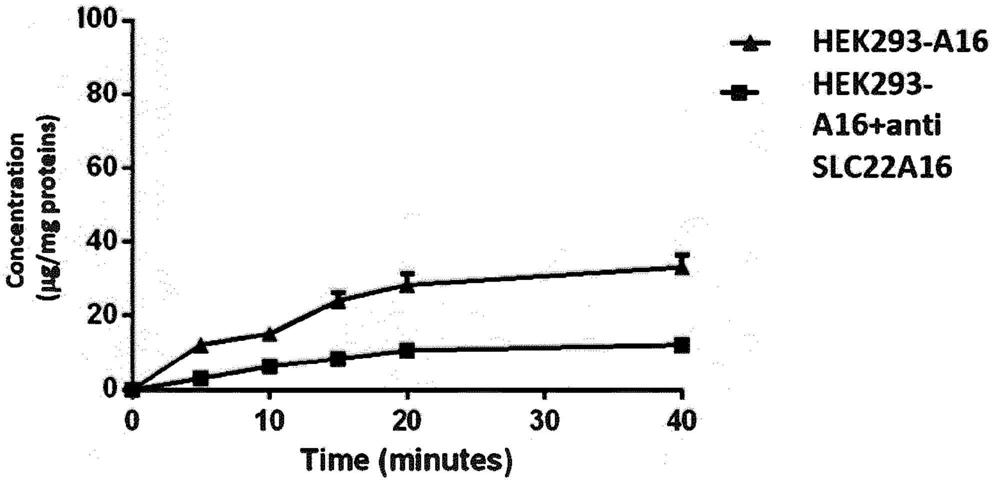

Figure 13

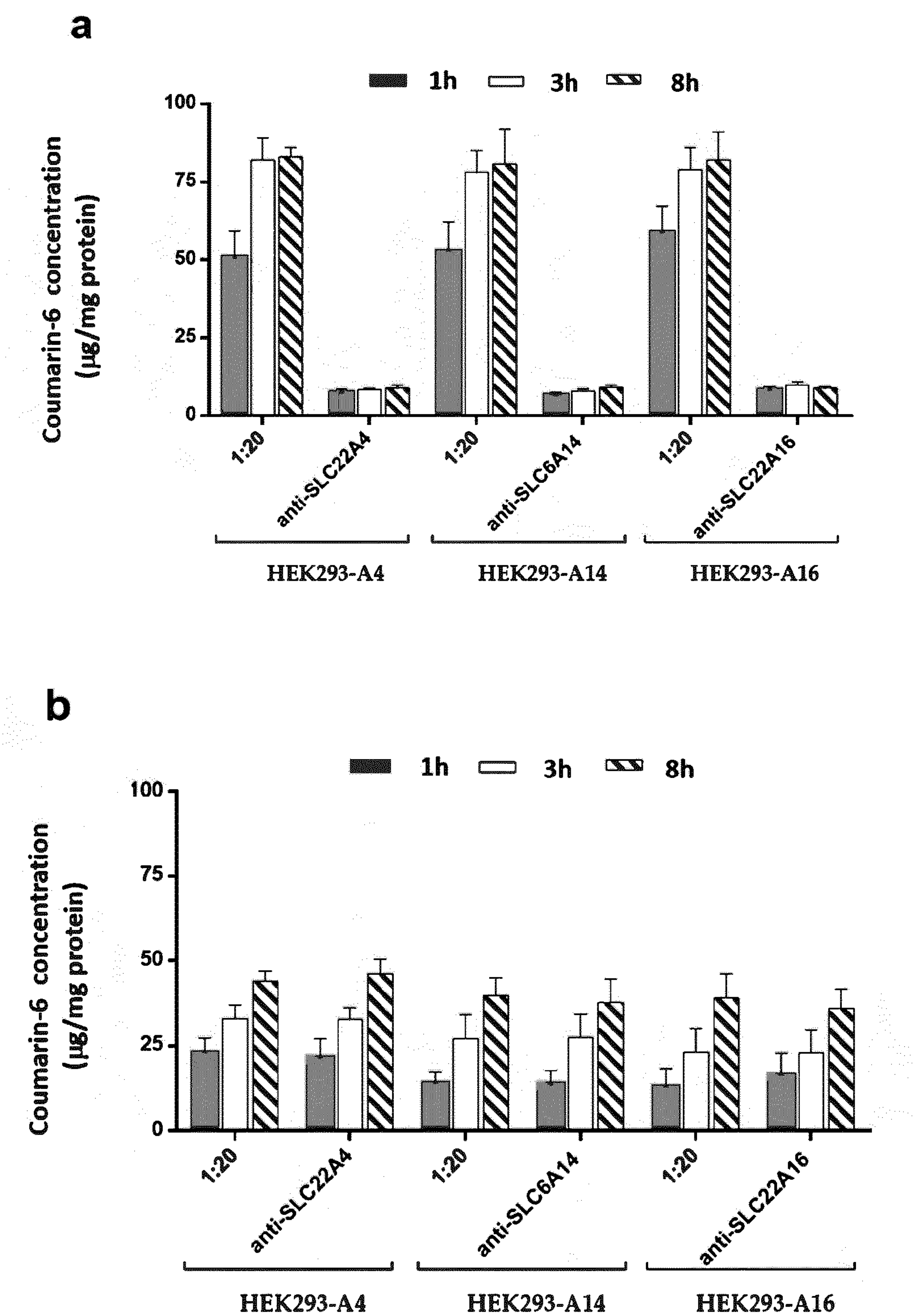
a
1h
3h
8h
Coumarin-6 concentration (µg/mg protein)
100
75
50
25
0
1:20
anti-SLC22A4
1:20
anti-SLC6A14
1:20
anti-SLC22A16
HEK293-A4
HEK293-A14
HEK293-A16
b
1h
3h
8h
Coumarin-6 concentration (µg/mg protein)
100
75
50
25
0
1:20
anti-SLC22A4
1:20
anti-SLC6A14
1:20
anti-SLC22A16
HEK293-A4
HEK293-A14
HEK293-A16

Figure 13 (continued)

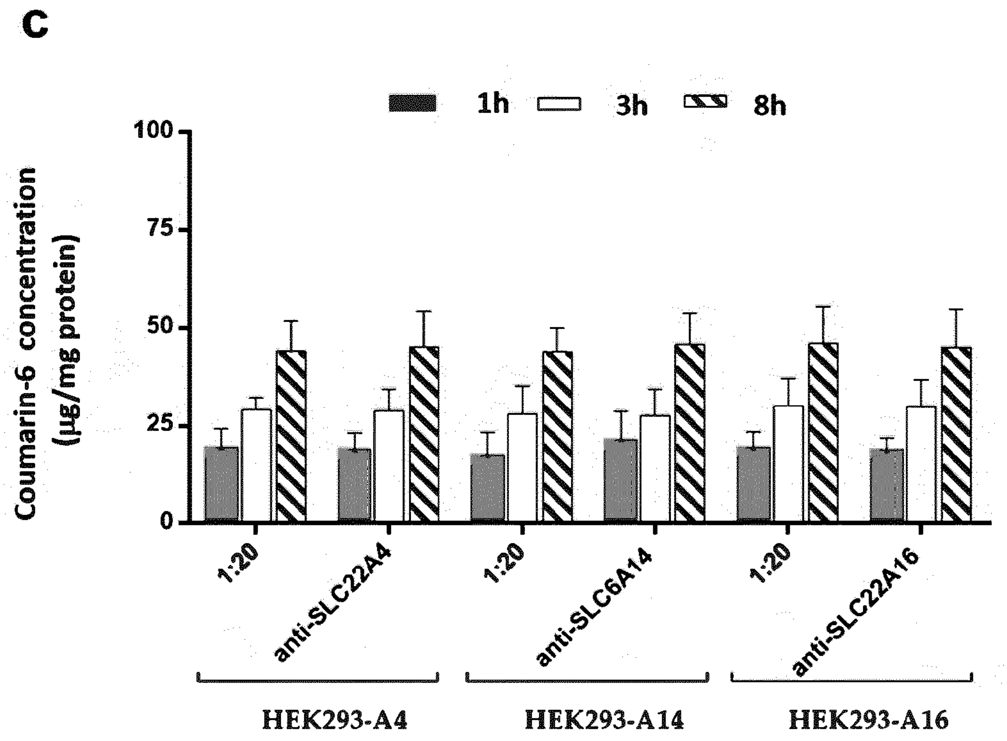

Figure 14

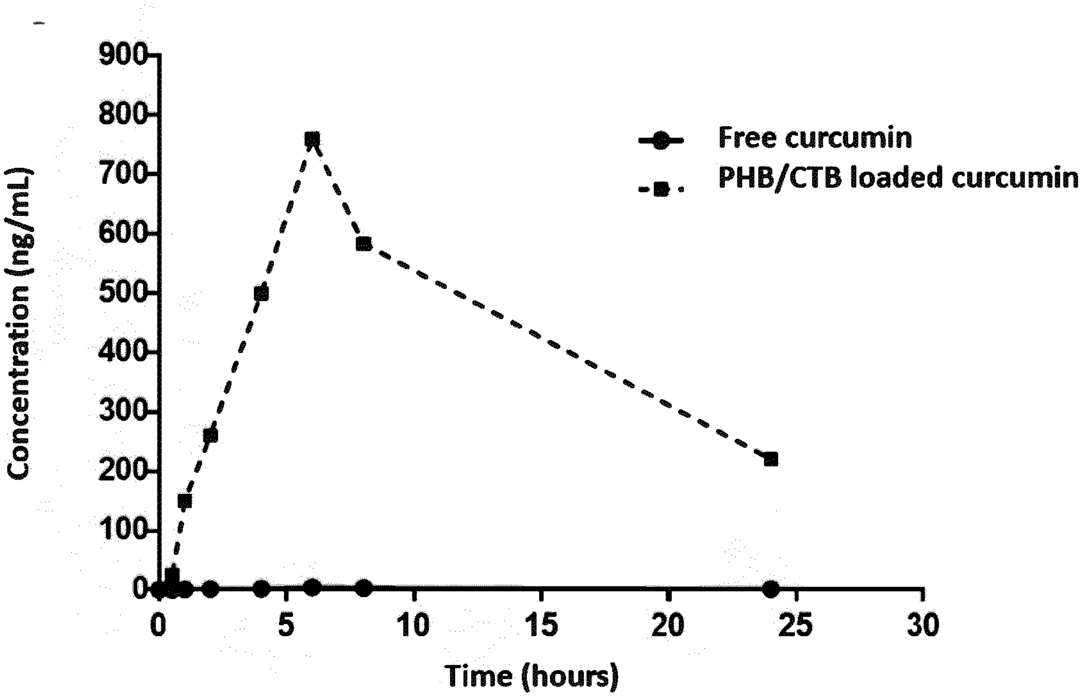
900
800
700
600
500
400
300
200
100
0
Concentration (ng/mL)
0
5
10
15
20
25
30
Time (hours)
Free curcumin
PHB/CTB loaded curcumin

Figure 15

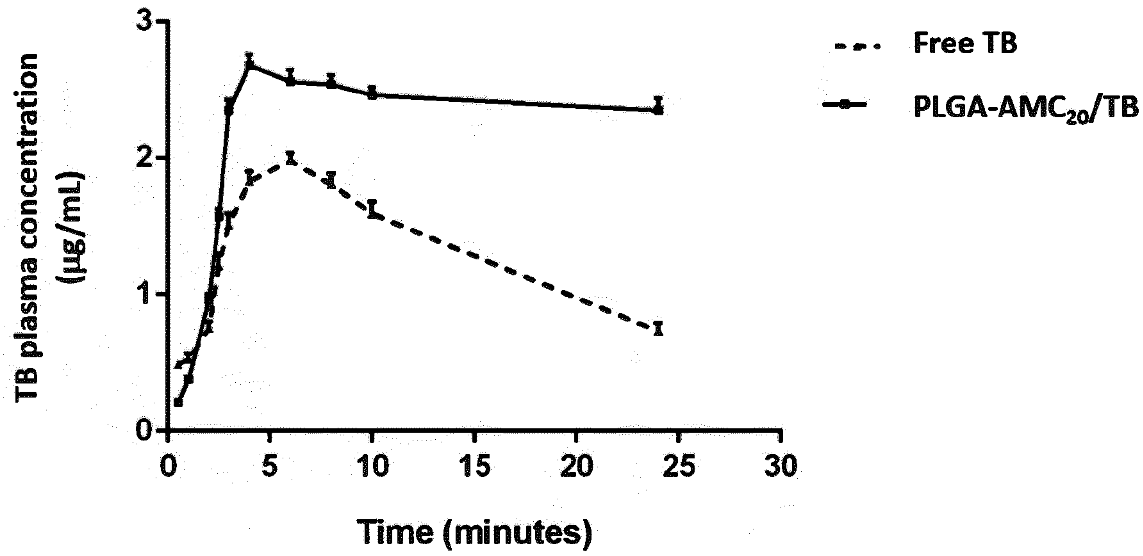
TB plasma concentration
(µg/mL)
0
1
2
3
0
5
10
15
20
25
30
Time (minutes)
Free TB
PLGA-AMC20/TB

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 107176911


### Non-patent literature cited in the description


- *Pharmaceutical Technology Europe,* 2005, vol. 17 (4), 21-28
- **LAROUI H. et al.** *Biomacromolecules,* 2007, vol. 8, 3879-3885
- **MAKHLOF A. et al.** *Eur J Pharm Biopharm,* 2009, vol. 72, 1-8
- **MEISSNER Y. et al.** *Int J Pharm,* 2006, vol. 316, 138-143
- **YANG H. et al.** *J Nanosci Nanotechnol,* 2009, vol. 9, 282-287
- **CHENG G et al.** *World J Gastroenterol,* 2004, vol. 10, 1769-1774
- **COUVREUR P ; VAUTHIER C.** *Pharmaceutical Research,* 2006, vol. 23, 1417-1450
- **SINGH M et al.** *Biotechnol Adv,* 2002, vol. 20 (5-6), 341-359
- **AVRVIZO, RR et al.** *Nano Lett.,* 2010, vol. 10, 2543-2548
- **ROTH M et al.** *British Journal of Pharmacology.,* 2012, vol. 165 (5), 1260-1287
- **GRÜNDEMANN D et al.** *Nature,* 1994, vol. 372, 549-552
- **KOEPSELL H.** *Mol Aspects Med,* 2013, vol. 34, 413-435
- **NIGAM K.S.** *Nature reviews Drug Discovery,* 2015, vol. 14, 29-44
- **KOEPSELL H et al.** *Pharm Res.,* July 2007, vol. 24 (7), 1227-51
- **WU Y. et al.** *Apoptosis,* 2015, vol. 20, 1099-108
- **COOTHANKANDASWAMY V. et al.** *J Pharmacol.,* 2016
- **OGURI T. et al.** *Biomed Rep.,* 2016, vol. 5, 639-643
- **MAILÄNDER V. ; LANDFESTER K.** *Biomacromolecules,* 2009, vol. 10, 2379-2400
- **MOREAU E et al.** *Drug, Target,* 2002, vol. 10, 161-173
- **BHATTARAI N et al.** *Adv Drug Deliv Rev.,* 2010, vol. 62, 83-99
- **PARK JH et al.** *Deliv Rev.,* 2010, vol. 62, 28-41
- **LV PP et al.** *Biomacromolecules,* 2011, vol. 12, 4230-4239
- **LONGFA KOU et al.** *Drug Delivery,* 2017, vol. 24 (1), 1338-1349
- **GAO Y et al.** *Int J Pharm,* 2009, vol. 371 (1-2), 156-162
- **WU et al.** *Experimental and Therapeutic Medicine,* 2017, vol. 14, 4153-4159
- **KAEWMOGUL et al.** *Am J Physiol Renal Physiol,* 2003, vol. 285, F1149-1159
- **SINGH M et al.** *Biotechnol Adv.,* 2002, vol. 20 (5-6), 341-359
- **D'AYALA G.G. et al.** *J Biomed Mater Res A.,* 2010, vol. 94, 619-30
- **CALARCO A. et al.** *The genotoxicity of PEIbased nanoparticles is reduced by acetylation of polyethylenimine amines in human primary cells.,* 2013, 10-7
- **FATHI M ; BARAR J.** *Perspectives highlights on biodegradable polymeric nanosystems for targeted therapy of solid tumors.,* 2017, 49-57
- **CALARCO et al.** *Toxicology Letters.,* 2013, vol. 218 (1), 10-17